# FASCICULE DE BREVET EUROPEEN

(11) **EP 2 688 902 B1**
(45) Date de publication et mention de la délivrance du brevet: **27.12.2017**
(21) Numéro de dépôt: 12717373.0
(22) Date de dépôt: 23.03.2012
(51) Int. Cl.: C07J 7/00, C07J 13/00, C07J 17/00, A61K 31/57, A61P 25/00, A61P 25/02, A61P 25/04, A61P 25/28

(54) **DERIVES DE L'ALLOPREGNANOLONE ET DE L'EPIALLOPREGNANOLONE ET LEURS UTILISATIONS POUR TRAITER UN ETAT NEUROPATHOLOGIQUE**
ALLOPREGNANOLON- UND EPIALLOPREGNANOLON-DERIVATE UND VERWENDUNGEN DAVON ZUR BEHANDLUNG EINES NEUROPATHOLOGISCHEN ZUSTANDES
DERIVATIVES OF ALLOPREGNANOLONE AND OF EPIALLOPREGNANOLONE AND USES THEREOF FOR TREATING A NEUROPATHOLOGICAL CONDITION

(30) Priorité: 23.03.2011 FR 1152390
(43) Date de publication de la demande: 29.01.2014
(73) Titulaire: Université de Strasbourg, 67000 Strasbourg (FR); Centre National de la Recherche Scientifique (C.N.R.S.), 75794 Paris Cedex 16 (FR)
(72) Inventeur: MENSAH-NYAGAN, Ayikoe Guy, F-67540 Otswald (FR); MEYER, Laurence, F-67000 Strasbourg (FR); PATTE-MENSAH, Christine, F-67540 Otswald (FR); TALEB, Omar, F-67200 Strasbourg (FR); MIESCH, Michel, F-67850 Herrlisheim (FR); GEOFFROY, Philippe, F-67100 Strasbourg (FR); RESSAULT, Blandine, F-37230 Fondettes (FR)
(74) Mandataire: Novagraaf Technologies
(86) Numéro de dépôt international: PCT/FR2012/050616
(87) Numéro de publication internationale: WO 2012/127176

(56) Documents cités:
- WO-A1-98/05337
- CZ-A3- 20 001 888
- SLAVIKOVA BARBORA ET AL: "3.ALPHA.-FLUORO ANALOGUES OF ALLOPREGNANOLONE AND THEIR BINDING TO GABAARECEPTORS", COLLECTION OF CZECHOSLOVAK CHEMICAL COMMUNICATIONS, INSTITUTE OF ORGANIC CHEMISTRY & BIOCHEMISTRY, PRAGUE; CZ, vol. 67, no. 1, 1 janvier 2002 (2002-01-01), pages 30-46, XP008073174, ISSN: 0010-0765, DOI: 10.1135/CCCC20020030

## Description

L'invention se rapporte à de nouveaux neurostéroides, notamment des dérivés de l'alloprégnanolone et de l'épialloprégnanolone, et ces dérivés comme médicaments pour le traitement de neuropathologies, en particulier les neuropathies induites par la chimiothérapie du cancer. Ces molécules peuvent avoir des effets aussi bien préventifs que curatifs.

Un des principaux effets indésirables du traitement du cancer qu'il soit chimiothérapique ou radiothérapique est l'induction d'une neuropathie périphérique douloureuse qui génère un inconfort chez les patients et complique sérieusement la prise en charge médicale du cancer.

On assiste, en particulier, à des dégénérescences périphériques diffuses particulièrement douloureuses provoquées, par exemple, par l'utilisation de certains médicaments anticancéreux ou antinéoplasiques tels que, entre autres, la vincristine, l'oxaliplatine et le taxol. L'efficacité de ces anticancéreux est donc fortement limitée par des effets secondaires dose-dépendants tels que les neuropathies périphériques provoquant des douleurs intenses chez environ 40% des patients.

Dans certains cas, la sévérité des symptômes est telle qu'elle nécessite le recours à des anticancéreux moins neurotoxiques mais peu efficaces contre le cancer ou à l'arrêt complet de la chimiothérapie, ce qui réduit considérablement l'espérance de vie des patients.

L'effet anti-tumoral des antinéoplasiques est basé sur leur capacité à perturber la polymérisation des monomères de tubulines en microtubules, ce qui inhibe la division cellulaire. Il a été suggéré que les neuropathies périphériques induites par les antinéoplasiques résulteraient de leur action toxique sur les microtubules des nerfs rachidiens ou crâniens conduisant à une détérioration du transport axonal et à une dégénérescence axonale. Les composés antinéoplasiques induisent également une démyélinisation axonale responsable de décharges électriques ectopiques, de paresthésies ou de douleurs neuropathiques.

Malheureusement, il n'existe pas de traitement prophylactique ou curatif efficace des symptômes neuropathiques sensorimoteurs provoqués par de nombreux médicaments anticancéreux utilisés en chimiothérapie.

Il est donc crucial de développer des stratégies pouvant contrecarrer les effets secondaires neuropathiques douloureux des médicaments anticancéreux pour assurer le confort des patients et améliorer l'efficacité des traitements.

Ainsi, il s'avère primordial d'identifier des composés neuroactifs capables de supprimer les neuropathies, en particulier les neuropathies induites par des médicaments anticancéreux tels que la vincristine, l'oxaliplatine ou le taxol, contribuant ainsi à l'amélioration des thérapies anticancéreuses en particulier celles basées sur l'utilisation des antinéoplasiques.

L'article publié dans le journal Neurobiology of Disease 30, (2008), p30-41 est relatif à la régulation du seuil de douleur provoquée par un stimulus thermique ou mécanique après une lésion du nerf sciatique. Chez des animaux soumis à une douleur neuropathique provoquée par une lésion du nerf sciatique, une injection intrathécale permettant d'augmenter la concentration d'alloprégnanolone ou 3alpha, 5alpha-THP (représentée ci-dessous) dans la moelle épinière induit une analgésie transitoire chez les rats neuropathiques. La 3alpha, 5alpha-THP est capable d'induire cette analgésie transitoire parce qu'elle potentialise l'inhibition centrale via l'activation du système GABAergique, le GABA étant le principal neurotransmetteur inhibiteur dans le système nerveux central des mammifères.
Le document WO98/05337 a pour objet un traitement du mal de tête de type migraine à base de dérivés de stéroïdes disubstitués en position 3 donc différents des dérivés selon l'Invention ou conformes à l'Invention qui sont monosubstitués en position 3.
Les effets neuroprotecteurs et/ou réparateurs du tissu nerveux des dérivés selon l'invention ou conformes à l'invention, découverts par la Demanderesse et qui peuvent permettre de contrer les symptômes douloureux des neuropathies périphériques (allodynie et hyperalgésie) provoqués par une thérapie anticancéreuse, ne sont pas mentionnés dans ce document de l'art antérieur.

Le document intitulé « 3alpha-fluoro analogues of allopregnanolone and their binding to GABAa receptors », collection of czechoslovak chemical communications, institute of organic chemistry & biochemistry, vol. 67, n°1, pages 30-46 traite spécifiquement de dérivés de l'Allopregnanolone qui interagissent avec les récepteurs GABAergiques de type a et qui modulent les dysfonctionnements liés au GABA. Selon ce document, les analogues d'AP peuvent moduler l'activité cellulaire via une modulation allostérique positive de ces récepteurs en présence de GABA.

Le document CZ20001888 concerne une molécule qui agit spécifiquement sur les récepteurs GABAa et les pathologies associées à la signalisation GABAergique.

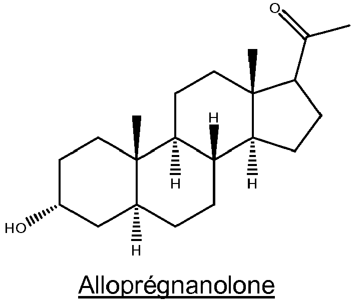

Le principe de stimulation de l'inhibition centrale, via le système GABAergique, ou de réduction de l'excitabilité neuronale, via les récepteurs opiacés comme le font la morphine et ses dérivés, est le mode d'action utilisé par de nombreux médicaments antalgiques qui permettent aux patients de ne plus ressentir la douleur alors que les altérations tissulaires et fonctionnelles responsables des influx nociceptifs persistent. Une fois que l'activité neuroinhibitrice de ces antalgiques classiques est terminée, les symptômes douloureux réapparaissent chez le patient : on parle alors d'analgésie transitoire. Ces symptômes douloureux persisteront tant que les dégâts tissulaires et fonctionnels ne seront pas réparés. C'est pour cette raison que de nombreux antalgiques utilisés actuellement restent inefficaces pour le traitement curatif des douleurs neuropathiques qui résultent le plus souvent de lésions traumatiques, diffuses, cytotoxiques ou d'un déséquilibre fonctionnel des structures nerveuses impliqués dans la nociception. En conséquence, les douleurs neuropathiques constituent un problème majeur de santé publique et la découverte de nouvelles stratégies qui permettraient l'éradication définitive de ces douleurs est un formidable enjeu médical et socio-économique. Pour ce faire, il apparait primordial de trouver des traitements capables de réparer les dommages ou altérations du tissu nerveux qui génèrent les signaux nocifs ou anormaux évocateurs de sensations douloureuses. Seule une réparation des dégâts tissulaires et neuronaux sera capable de restaurer une perception sensorielle normale et de supprimer définitivement la douleur neuropathique.

La demanderesse a maintenant trouvé, de façon tout à fait surprenante, des dérivés d'alloprégnanolone et d'épialloprégnanolone qui ont tous en commun, avec des degrés d'efficacité variables, une propriété neuroprotectrice, en particulier la capacité de réparer le tissu nerveux endommagé et d'éradiquer par conséquent la douleur neuropathique de manière définitive. Par ailleurs, les dérivés d'alloprégnanolone et d'épialloprégnanolone caractérisés par la demanderesse sont également capables de protéger des cultures de cellules nerveuses contre la mort induite par le stress oxydant, ce qui souligne le fort potentiel thérapeutique de ces dérivés pour le traitement des pathologies neurodégénératives résultant d'une anomalie du métabolisme oxydatif.

En particulier, la Demanderesse a trouvé que les dérivés O-allyl de l'épialloprégnanolone, avantageusement la 1-((3S,5S,10S,13S,17S)-3-(allyloxy)-10,13-dimethylhexadecahydro-1H-cyclopenta[a]phenanthren-17-yl)ethanone, s'avèrent être des analgésiques capables de supprimer définitivement les symptômes douloureux, tels que l'allodynie et l'hyperalgie, provoqués par exemple par les anticancéreux sans induire de prolifération cellulaire qui est contre-indiquée chez les patients cancéreux. En outre, grâce à leur capacité de réparation ou de protection contre les dommages tissulaires et fonctionnels causés par les anticancéreux sur le système nerveux périphérique, ces neurostéroïdes exercent un puissant effet neuroprotecteur.

Quant aux dérivés O-allyl de l'alloprégnanolone, en particulier la 1-((3R,5S,10S,13S,17S)-3-(allyloxy)-10,13-diméthylhexadecahydro-1H-cyclopenta[a]phenanthren-17-yl)ethanone, aux dérivés 12-oxo-alloprégnanolone, en particulier la (3R,5S,10S,13S,17S)-17-acétyl-3-hydroxy-10,13-dimethyltetradecahydro-1H-cyclopenta[a]phenanthren-12(2H)-one et aux dérivés 12-oxo-épiallopregnanolone, en particulier la (3S,5S,10S,13S,17S)-17-acetyl-3-hydroxy-10,13-dimethyltetradecahydro-1H-cyclopenta[a]phenanthren-12(2H)-one, la Demanderesse a trouvé que ce sont des neuroprotecteurs qui induisent également une prolifération cellulaire. Par conséquent, ces molécules sont contre-indiquées pour le traitement des neuropathies induites par la chimiothérapie du cancer. En revanche, elles sont tout à fait indiquées chez le sujet non cancéreux et peuvent s'avérer bénéfiques pour le traitement des pathologies neurodégénératives telles que la maladie d'Alzheimer, la maladie de Parkinson, la sclérose latérale amyotrophique, etc... En effet, grâce à leur activité neuroproliférative, particulièrement élevée pour la 1-((3R,5S,10S,13S,17S)-3-(allyloxy)-10,13-dimethylhexadecahydro-1H-cyclopenta[a]phenanthren-17-yl)ethanone et la (3R,5S,10S,13S,17S)-17-acetyl-3-hydroxy-10,13-dimethyltetradecahydro-1H-cyclopenta[a]phenanthren-12(2H)-one, ces neurostéroïdes peuvent stimuler ou réactiver la neurogenèse dans les structures du cerveau adulte comme le gyrus dentelé de l'hippocampe et les zones sous-ventriculaires qui contiennent des cellules souches neurales afin de compenser les pertes neuronales observées chez les patients atteints de telles maladies neurodégénératives.

L'invention vise donc un dérivé de l'alloprégnanolone ou de l'épi-alloprégnanolone de formule (I) suivante : 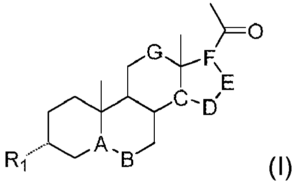 dans laquelle
- **R¹** est un groupement choisi parmi les groupements 3-alpha ou 3-beta hydroxy, les groupements 3-alpha ou 3-beta O-allyl, les groupements 3-alpha ou 3-beta O-propargyl, les groupements 3-alpha ou 3-beta O-glycol, les groupements 3-alpha ou 3-beta O-PEG, les groupements 3-alpha ou 3-beta O-glycol-allyl et les groupements 3-alpha ou 3-beta O-PEG-allyl,
- **A** est un atome de carbone substitué par un atome choisi parmi le 5-H alpha et le 5-H beta et **B** est un groupement méthylène ; ou
   **A** et **B** sont des atomes de carbone formant une double liaison 5-6 ;
- **C** est un atome de carbone substitué par un atome choisi parmi le 14-H alpha, le 14-H beta, le groupement 14-alpha OH et le groupement 14-beta OH et **D** est un groupement méthylène ; ou
   **C** et **D** sont des atomes de carbone formant une double liaison 14-15;
- **F** est un atome de carbone substitué par un atome choisi parmi le 17-H alpha et le 17-H beta et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
   **F** est un atome de carbone substitué par un groupement choisi parmi le 17-alkyl-alpha, le 17-alkyl-beta, le 17-OR²-alpha et le 17-OR²-beta, avec R² un groupement choisi parmi le allyl, O-propargyl, glycol, PEG, glycol-allyl, PEG-allyl, et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
   **E** et **F** sont impliqués dans un cycle époxy ou un cyclopropyle et sont choisis parmi le 16, 17-époxy-alpha, le 16, 17-époxy-beta, le 16, 17-méthylène-alpha et le 16, 17-méthylène-beta ; ou
   **E** et **F** sont des atomes de carbone formant une double liaison 16-17 ;
- **G** est un carbonyle, un méthylène ou un atome de carbone substitué par un groupement 12-OR³ alpha ou 12-OR³ beta avec R³ un atome d'H ou un groupement choisi parmi les groupements acétyle, alkyle et aryle, R³ pouvant être choisi parmi les alkyl_{C1-C6}, le benzyle, le p-méthoxybenzyle, le benzoyle, le tigloyle, l'angeloyle, le 2,2,2-trichloroéthoxycarbonyle, le o-aminobenzoyle, le nicotinoyle, le 2-méthylbutyryle, l'isovaleryle, le cinnamoyle, le coumaroyle, le o-hydroxybenzoyle et l'anthraniloyle, à l'exclusion de (i) la molécule de formule BR053 suivante : 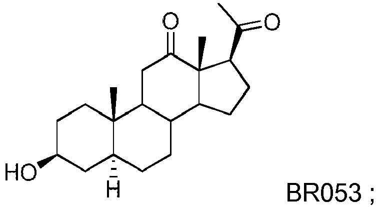 et
   de (ii) la molécule de formule BR338 suivante :

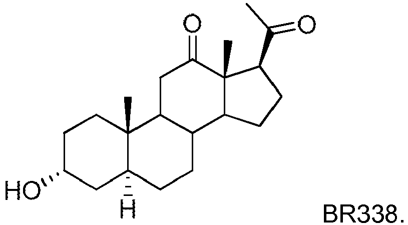

Selon un mode de réalisation, ce dérivé est caractérisé en ce que :
- **R¹** est un groupement choisi parmi les groupements 3-alpha ou 3-beta O-allyl, les groupements 3-alpha ou 3-beta O-propargyl, les groupements 3-alpha ou 3-beta O-glycol, les groupements 3-alpha ou 3-beta O-PEG, les groupements 3-alpha ou 3-beta O-glycol-allyl et les groupements 3-alpha ou 3-beta O-PEG-allyl,
- **A** est un atome de carbone substitué par un atome choisi parmi le 5-H alpha et le 5-H beta et **B** est un groupement méthylène ; ou
   **A** et **B** sont des atomes de carbone formant une double liaison 5-6 ;
- **C** est un atome de carbone substitué par un atome choisi parmi le 14-H alpha, le 14-H beta, le 14-alpha OH et le 14-beta OH et **D** est un groupement méthylène ; ou
   **C** et **D** sont des atomes de carbone formant une double liaison 14-15 ;
- **F** est un atome de carbone substitué par un atome choisi parmi le 17-H alpha et le 17-H beta et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
   **F** est un atome de carbone substitué par un groupement choisi parmi le 17-alkyl-alpha, le 17-alkyl-beta, le 17-OR²-alpha et le 17-OR²-beta, avec R² un groupement choisi parmi le allyl, propargyl, glycol, PEG, glycol-allyl, PEG-allyl, et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
   **E** et **F** sont impliqués dans un cycle époxy ou un cyclopropyle et sont choisis parmi le 16, 17-époxy-alpha, le 16, 17-époxy-beta, le 16, 17-méthylène-alpha et le 16, 17-méthylène-beta ; ou
   **E** et **F** sont des atomes de carbone formant une double liaison 16-17 ; et
- **G** est un carbonyle, un méthylène ou un atome de carbone substitué par un groupement 12-OR³ alpha ou 12-OR³ beta avec R³ un atome d'H ou un groupement choisi parmi les groupements acétyle, alkyle et aryle,
R³ pouvant être choisi parmi les alkyl_{C1-C6}, le benzyle, le p-méthoxybenzyle, le benzoyle, le tigloyle, l'angeloyle, le 2,2,2-trichloroéthoxycarbonyle, le o-aminobenzoyle, le nicotinoyle, le 2-méthylbutyryle, l'isovaleryle, le cinnamoyle, le coumaroyle, le o-hydroxybenzoyle et l'anthraniloyle.

Selon un mode de réalisation, ce dérivé est caractérisé en ce qu'il s'agit d'un dérivé de formule (I) dans laquelle **R¹** est un groupement choisi parmi les groupements 3-beta O-allyl, 3-beta O-propargyl, 3-beta O-glycol, 3-beta O-PEG, 3-beta O-glycol-allyl et 3-beta O-PEG-allyl et /ou **G** est un méthylène.

On peut citer comme dérivés selon l'invention :
- le dérivé de formule BR297 suivante : 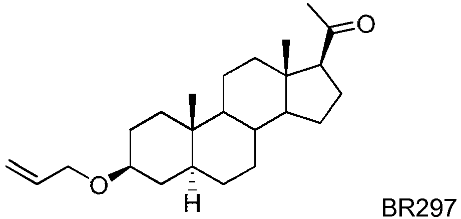 et
- le dérivé de formule BR351 suivante :

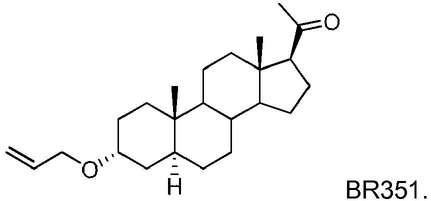

Les dérivés selon l'invention ou conformes à l'invention, en particulier les composés BR053, BR338, BR297 et BR351, peuvent trouver leur utilisation comme médicament. L'invention a donc aussi pour objet l'utilisation des dérivés selon l'invention ou conformes à l'invention pour l'obtention d'un médicament.

Ainsi les dérivés selon l'invention définis ci-dessus ou le dérivé de formule BR338 ou de formule BR053, définies ci-dessus, s'avèrent utiles comme agent neuroprotecteur et/ou comme agent stimulant la prolifération des cellules nerveuses. Ces composés s'avèrent, en particulier, utiles pour le traitement d'une neuropathologie, ladite neuropathologie pouvant être choisie parmi les neuropathies périphériques et centrales, la douleur chronique, les maladies neurodégénératives et les déficits sensorimoteurs induits par la neuroinflammation. De préférence, la neuropathologie est choisie parmi les neuropathies périphérique et centrale.

La neuropathologie peut être liée à une allodynie ou à une hyperalgésie.

La neuropathie peut être induite par un traitement anticancéreux, en particulier un traitement chimiothérapique mettant en oeuvre un antinéoplasique choisi, par exemple, parmi les poisons du fuseau, les agents intercalants, les inhibiteurs des DNA topoisomérases, les inhibiteurs de la tyrosine kinase, les inhibiteurs des mTOR, les anticorps monoclonaux anti-angiogènes, les inhibiteurs de la synthèse et de la réplication de l'ADN et les inhibiteurs de la polymérisation ou de la dépolymérisation des microtubules, encore plus avantageusement choisi parmi la vincristine, l'oxaliplatine, le docétaxel, le paclitaxel et les dérivés du taxol en général.

En particulier, les dérivés selon l'invention définis ci-dessus ou en particulier les dérivés de formule BR297 et BR351, définies ci-dessus, ou les dérivés de formules BR338 et BR053, définies ci-dessus, s'avèrent utiles comme neuroprotecteurs, en particulier comme neuroréparateurs. Cet aspect de neuro-réparation est primordial pour traiter efficacement toutes les pathologies provoquées par des lésions du tissu nerveux.

Les dérivés selon l'invention, à l'exclusion de BR297, et en particulier le dérivé de formule BR351 définie ci-dessus ou les dérivés de formule BR338 ou de formule BR053, définies ci-dessus sont particulièrement utiles comme agents stimulant la prolifération des cellules nerveuses. Cet aspect neuro-prolifératif est à éviter pour le traitement anticancéreux afin de ne pas stimuler la prolifération des cellules cancéreuses. Par contre, il s'avère primordial pour combler le déficit en cellules neuronales causé par une pathologie neurodégénérative ou par un accident avec lésion d'un tissu nerveux sain.

Les dérivés selon l'invention ou le dérivé de formule BR338 ou de formule BR053 sont utiles pour prévenir la mort cellulaire des neuroblastomes induite par le stress oxydant.

Dans le cadre de la présente invention, on entend par :
- « effet neuroprotecteur », le fait (i) d'empêcher la mort des cellules nerveuses suite à une cytotoxicité ou à un stress provoqué par des facteurs endogènes ou exogènes, (ii) de favoriser la réparation des neurones et du tissu nerveux dans le cas d'une lésion, accidentelle ou non, voire provoquée par un traitement curatif, tel qu'une chimiothérapie, et/ou (iii) de réactiver la neurogenèse dans le système nerveux mature ou de favoriser la prolifération des cellules nerveuses afin de combler un déficit cellulaire causé par une maladie neurodégénérative.
- « effet neuroprotecteur », le fait de favoriser la réparation des neurones et du tissu nerveux dans le cas d'une lésion, accidentelle ou non, voire provoquée par un traitement curatif, tel qu'une chimiothérapie.
- « effet neuroprolifératif », le fait de réactiver la neurogenèse dans le système nerveux mature ou de favoriser la prolifération des cellules nerveuses afin de combler un déficit cellulaire causé par une maladie neurodégénérative.
- « effet antinociceptif », le fait de bloquer la transmission des messages nocifs de la périphérie vers le système nerveux central ou d'inhiber la perception des stimulations utilisées pour produire la douleur chez des sujets sains ou pathologiques.
- « effet analgésique » le fait de supprimer la douleur existant chez des sujets pathologiques. L'existence de douleur est liée aux facteurs étiologiques de la pathologie.
- « hyperalgie », une sensation douloureuse d'intensité anormalement élevée à la suite d'une stimulation douloureuse.
- « allodynie », une sensation douloureuse suscitée par un stimulus qui n'est normalement pas ressenti comme douloureux.
- « neuropathologie », l'ensemble des troubles fonctionnels et des altérations morphologiques macroscopiques et microscopiques tissulaires ou cellulaires observés dans les maladies du système nerveux central et périphérique. Quelques exemples de neuropathologies sont les neuropathies périphérique et centrale, la douleur chronique, les maladies neurodégénératives (maladie d'Alzheimer, sclérose latérale amyoptrophique, Chorée de Huntington, maladie de Parkinson), les pathologies neuroinflammatoires et/ou autoimmunes (sclérose en plaque, Encephalite Autoimmune).
- « neuropathie », l'ensemble des affections du système nerveux périphérique, c'est-à-dire des nerfs moteurs et sensitifs des membres, mais également des nerfs du système neurovégétatif ou autonome contrôlant l'activité des organes et tissus non squelettiques. Les neuropathies périphériques comprennent, entre autres la polynévrite, la multinévrite ou la mononévrite, la polyradiculonévrite chronique, les maladies de Charcot-Marie-Tooth. Dans certains cas, les lésions provoquant les dysfonctionnements sensori-moteurs et/ou neurovégétatifs caractéristiques des neuropathies ne sont pas localisées sur les nerfs (système nerveux périphérique) mais se trouvent dans le cerveau ou la moelle épinière : on parle alors de neuropathie centrale.
- « antinéoplasique », un médicament utilisé pour détruire les cellules cancéreuses ou empêcher leur prolifération. On peut citer comme anticancéreux : les poisons du fuseau, les agents intercalants, les inhibiteurs des DNA topoisomérases, les inhibiteurs de la tyrosine kinase, les inhibiteurs des mTOR, les anticorps monoclonaux anti-angiogènes, les inhibiteurs de la synthèse et de la réplication de l'ADN et les inhibiteurs de la polymérisation ou de la dépolymérisation des microtubules. On peut en particulier citer la vincristine, l'oxaliplatine, le docétaxel et la paclitaxel.
- « groupe (Cₓ-Cₜ) », un groupe comprenant entre x et t atomes de carbone.
- « groupe alkyle », un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl, cyclopropylméthyl.
- « groupe aryle », un groupe aromatique mono ou bicyclique comportant de 6 à 10 atomes. A titre d'exemples de groupes aryles, on peut citer le phényle et le naphthyle.

L'invention concerne des dérivés d'alloprégnanolone et d'épi alloprégnanolone.

La synthèse en particulier des dérivés de l'O-allyl epi-allo-prégnanolone et l'O-allyl alloprégnanolone est représentée ci-dessous sur le schéma 1 suivant. Toutefois la synthèse des autres dérivés selon l'invention peut être facilement obtenue en prenant comme composé de départ des composés facilement synthétisables par l'homme du métier et/ou disponibles dans le commerce.

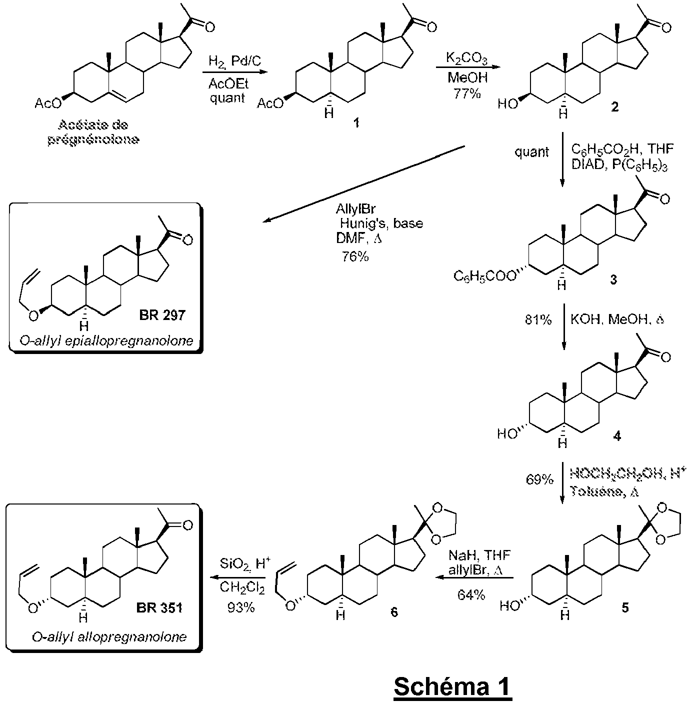

Le schéma 1 ci-dessus représente les différentes étapes permettant d'obtenir l'O-allyl epi-allo-prégnanolone et l'O-allyl alloprégnanolone à partir d'acétate de prégnénolone.

Dans un premier temps, la double liaison de l'acétate de prégnénolone disponible dans le commerce est réduite. Une hydrogénation stéréospécifique de la double liaison 5,6 de l'acétate de prégnénolone peut être réalisée en présence d'une quantité catalytique de Pd/C pour fournir le composé **1.** Puis une étape de déprotection du groupement 3 beta-acétate a lieu. Cette étape de déprotection peut avoir lieu avec du carbonate de potassium dans du méthanol. A l'issue de cette réaction de déprotection, on obtient l'epi-alloprégnanolone **2.** Ce composé epi-alloprégnanolone **2** peut ensuite être transformé en dérivé O-allyl epi-alloprégnanolone **BR 297.** Cette réaction peut se faire par le traitement de l'epi-alloprégnanolone **2** avec du bromure d'allyl en présence de la base de Hünig (N, N-diisopropylethylamine) dans du diméthylformamide à reflux.

Mais l'epi-alloprégnanolone **2** peut aussi être à l'origine de la synthèse d'un autre dérivé de prégnénolone selon l'invention: le O-allyl alloprégnanolone **BR351.**

Dans ce cas, cette synthèse peut se faire selon les réactions suivantes. L'epi-alloprégnanolone **2** peut être soumis à une réaction de Mitsunobu pour former le 3-alpha-benzoate-alloprégnanolone **3.** S'en suit une réaction de déprotection du groupement 3-alpha- benzoate avec de l'hydroxyde de potassium dans du méthanol à reflux. Cette réaction de déprotection fournit le composé alloprégnanolone **4.** Une réaction de protection du groupe carbonyle dudit alloprégnanolone **4** est ensuite effectuée. Cette réaction peut s'effectuer avec, par exemple, du glycol d'éthylène en présence d'une quantité catalytique de p-TsOH. Cette réaction de protection donne ainsi lieu à la formation d'un composé **5.** Ce composé **5** peut ensuite subir une réaction d'allylation par traitement avec de l'hydrure de sodium et du bromure d'allyl dans du THF à reflux. On obtient alors le O-allyl alloprégnanolone protégé **6,** qui une fois déprotégé, par exemple par de la silice acidifiée, conduit au composé O-allyl alloprégnanolone **BR 351.**

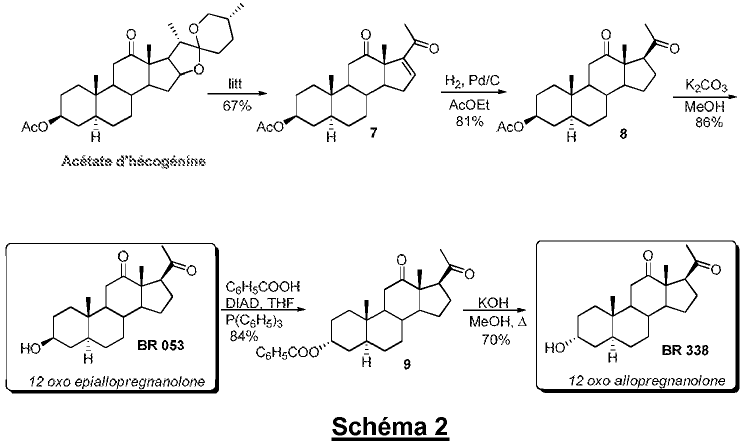

Le schéma 2 ci-dessus représente différentes étapes permettant d'obtenir la 12-oxo-epialloprégnanolone et la 12-oxo-alloprégnanolone à partir d'acétate d'hécogenine.

La synthèse du composé 12-oxo-epialloprégnanolone BR053 peut être réalisée par la transformation du groupement dioxaspiro de l'acétate d' hécogenine disponible commercialement. Pour ce faire, l'acétate d' hécogenine est tout d'abord traité dans l'anhydride acétique par de la pyridine et en présence de NH₄Cl. Le produit ainsi obtenu est oxydé à l'aide de trioxyde de chrome dans l'acide acétique pour donner après chauffage dans l'acide acétique le composé **7.**

Une hydrogénation de l'énone **7** obtenue suivie par la déprotection du groupement 3 beta-acétate pouvant être effectué avec du carbonate de potassium dans le méthanol conduit au composé 12-oxo-epialloprégnanolone **BR053**

Le composé 12-oxo-epialloprégnanolone **BR053** peut ensuite être soumis à une réaction de Mitsunobu conduisant au composé **9** qui après déprotection du groupement 3-alpha- benzoate pouvant être effectué par de l'hydroxyde de potassium dans le méthanol à reflux mène au composé 12-oxo-alloprégnanolone **BR 338.**

La présente Invention concerne des dérivés de l'alloprégnanolone ou de l'épiprégnanolone qui engendrent principalement et clairement un effet cellulaire totalement indépendant des récepteurs GABAa. En effet, des expériences électrophysiologiques pratiquées sur des cellules de neuroblastomes humains via une technique de Patch-Clamp, dans une configuration cellule-attachée de Patch-Clamp, ont permis d'observer que lorsque le GABA est absent de la pipette, i.e. non-introduit, ou quand les récepteurs GABAa ne sont pas activés, lesdits dérivés étaient capables d'induire une activité cellulaire spécifique. Ces résultats indiquent que les dérivés selon l'invention ou conformes à l'invention interagissent avec d'autres canaux ioniques et des récepteurs différents des récepteurs GABAa.
Les dysfonctionnements des récepteurs GABAa ne sont pas la principale cause des neuropathies douloureuses engendrées par une chimiothérapie. L'attaque neurotoxique des médicaments anticancéreux engendrant des neuropathies douloureuses est principalement ciblée contre des éléments structuraux tels que le cytosquelette, les neurofilaments, les transporteurs, les protéines calcium-dépendantes du ganglion spinal et/ou des axones des nerfs périphériques ainsi que les protéines clés dans la fabrication de la gaine de myéline.
En conséquence, tous les essais qui portent exclusivement ou principalement sur la modulation des récepteurs GABAa sont inefficaces dans l'éradication des neuropathies douloureuses induites par les antinéoplasiques.
Ainsi, les propriétés sophistiquées des dérivés selon l'invention ou conformes à l'invention, en particulier du BR297, constituent un moyen pharmacologique original et puissant afin de contrer efficacement les symptômes douloureux neuropathiques engendrés par une chimiothérapie. En effet, le BR297, qui peut essentiellement activer des cibles cellulaires différentes de celles des récepteurs GABAa afin de prévenir ou réparer les dommages neuronaux/axonaux engendrés par les antinéoplasiques, peut également exercer une modulation allostérique partielle du système GABAergique afin de renforcer ses effets bénéfiques.

Les molécules selon l'invention ou conformes à l'invention sont efficaces tant en tant que traitement préventif que curatif, leur principal atout étant leur capacité thérapeutique des neuropathies au lieu de ne cibler, comme les molécules de l'art antérieur, que les symptômes nociceptifs.

D'autres avantages pourront encore apparaître à l'homme du métier à la lecture des exemples ci-dessous. Les exemples suivants sont donnés à titre illustratif et ne sont pas limitatifs.

### EXEMPLES

Les points de fusion ont été mesurés sur un appareil de point de fusion de Stuart Scientific (SMP 3) et sont non corrigés. Les réactions ont été effectuées sous l'argon avec des solvants dégazés sous agitation magnétique.

L'Et₂O et le THF ont été distillés en présence de Na/benzophenone.

La chromatographie en couche mince (TLC) a été effectuée sur des plaques de gel de silice (Merck 60F254) et les spots ont été visualisées sous lampe UV (254 ou 365 nm) et/ou pulvérisées avec une solution d'acide phosphomolybdique (25 g d'acide phosphomolybdique, 10 g de cérium sulfate, 60 ml d'H₂SO4, 940 ml d'H2O) suivi sur une plaque chaude en chauffant.

Pour la chromatographie sur colonne, du gel de silice (Merck Si 60 40-60 µm) a été utilisé.

Des spectres IR ont été enregistrés sur spectrophotomètre Bruker Alpha (ATR, cm⁻¹). Des spectres RMN ¹H ont été enregistrés à 300 MHz (Bruker AC-300) et des spectres RMN ¹³C à 75 MHz (Bruker AC-300) utilisant le signal du solvant non deuteré résiduel comme référence interne. Des données RMN ¹H significatives sont tabulées dans l'ordre suivant : déplacement chimique (δ) exprimé en ppm, multiplicité (s, singulet; d, doublet; t, triplet; q, quartet; m, multiplet), couplant des constantes en hertz, nombre de protons.

Des spectres de masse haute résolution (HRMS) ont été exécutés sur un Agilent 6520 Masse Accurate Q-TOF.

### Exemple 1 : Synthèse du (3S,5S,10S,13S,17S)-17-acetyl-10,13-dimethylhexadecahydro-1H-cyclopenta[a]phenanthren-3-yl acetate 1

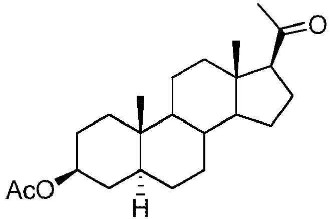

À une solution d'acétate de prégnénolone (5.14 g, 14.3 mmol) dans AcOEt (125 ml), a été ajouté Pd/C à 10 % (0.48 g). Le mélange réactionnel a été ventilé sous H₂ (sous vide/H₂) et a été agité vigoureusement sous H₂ jusqu'à l'absorption de 1.1 eq de H₂ (375 ml, 15.7 mmol). Le mélange réactionnel a été ventilé avec l'Argon (sous vide/argon), le catalyseur a été filtré sur Celite et lavé avec EtOH (100 ml), AcOEt (100 ml), CH₂Cl₂ (100 ml) puis concentré sous pression réduite. Le solide obtenu a été purifié par chromatographie (100 g SiO₂, éther de pétrole / acétate d'éthyle, 80/20) fournissant le composé 1 (4.98 g, 96 %, F : 145.2-146.8°C) ayant l'aspect d'un solide blanc.
**IR (ATR):** 1727, 1705.
**¹H NMR (CDCl₃):** 4.67 (1H, tt, J = 5.0-11.3 Hz, H₃); 2.50 (1H, t, J = 8.8 Hz, H₁₇); 2.20-1.90 (2H, m); 2.09 (3H, s, H₂₁); 2.00 (3H, s, OAc); 1.90-0.75 (19H, m); 0.80 (3H, s, Me); 0.68 (1H, td, J = 4.2-12.0 Hz); 0.58 (3H, s, Me).
**¹³C NMR (CDCl₃):** 209.3 (C=O); 170.4 (C=O, OAc); 73.4 (CH, C₃), 63.7 (CH, C₁₇); 60.2 (CH₂); 56.5 (CH); 54.0 (CH); 44.5 (CH); 44.0 (CH); 38.9 (CH₂); 35.4 (CH); 35.4 (C); 33.9 (CH₂); 31.8 (CH₂); 28.4 (CH₂); 27.3 (CH₂); 24.3 (CH₂); 22.7 (CH₂); 21.1 (CH₂); 20.9 (CH₃); 14.1 (CH₃); 13.3 (CH₃); 12.1 (CH₃).

### Exemple 2: Synthèse du 1-((3S,5S,10S,13S,17S)-3-hydroxy-10,13-dimethylhexadecahydro-1H cyclopenta[a]phenanthren-17-yl)ethanone 2

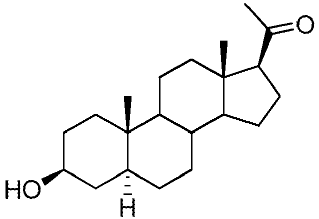

À une solution de composé 1 (4.82 g, 13.4 mmol) dans un mélange de MeOH/THF (1/1, 100 ml) a été ajouté du K₂CO₃ (1.97 g, 14.3 mmol). Le mélange réactionnel a été agité 4 h à température ambiante. L'eau (100 ml) a été ajoutée et la phase aqueuse a été extraite avec CH₂Cl₂ (2 x 100 ml) et avec AcOEt (1 x 100 ml). Les phases organiques combinées ont été lavées avec de la saumure (1 x 100 ml), séchées sur Na₂SO₄, filtrées et concentrées sous pression réduite. La matière brute a été purifiée par chromatographie (150 g SiO₂, éther de pétrole / acétate d'éthyle, 6:4) fournissant le composé 2 (3.90 g, 91 %, mp: 192.3-195.0°C) ayant l'aspect d'un solide blanc.
**IR (ATR):** 3385, 1698, 1682.
**¹H NMR (CDCl₃):** 3.63- 3.50 (1H, m, H₃); 2.50 (1H, t, J= 9Hz, H₁₇); 2.30-1.95 (2H, m); 2.09 (3H, s, H₂₁); 1.90- 1.55 (8H, m); 1.50-0.75 (11H, m); 0.79 (3H, s, Me); 0.66 (1H, td, J= 3.9-12.0 Hz); 0.58 (3H, s, Me).
**¹³C NMR (CDCl₃):** 209.7 (C=O); 71.2 (CH, C₃); 63. (CH); 567 (CH); 54.2 (CH); 44.8 (CH); 44.2 (C); 39.2 (CH₂); 38.1 (CH₂); 37.0 (CH₂); 35.5 (CH + C); 32.0 (CH₂); 31.5 (CH₃); 31.4 (CH₂); 28.6 (CH₂); 24.4 (CH₂); 22.8 (CH₂); 21.2 (CH₂); 13.4 (CH₃); 12.3 (CH₃).

### Exemple 3: Synthèse de la 1-((3S,5S,10S,13S,17S)-3-(allyloxy)-10,13-dimethylhexadecahydro-1H-cyclopenta[a]phenanthren-17-yl)ethanone BR297

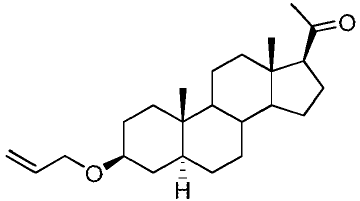

La N,N-diisopropylethylamine (5.10 ml, 29.5 mmol) a été ajoutée à une solution de composé 2 (1.85g, 5.8 mmol) dans du DMF (30 ml) et le mélange réactionnel a été agité sous reflux pendant 30 min. Du bromure d'allyle (1.50 ml, 17.3 mmol) a été ajouté à température ambiante et le mélange réactionnel a été chauffé sous reflux pendant 6 h 30. Le solvant a été évaporé sous pression réduite. Le résidu a été dissout dans de l'AcOEt (20 ml) et lavé à l'eau (2 x 20 ml). Les phases aqueuses combinées ont été extraites avec de l'AcOEt (2 x 20 ml). Les phases organiques combinées ont été lavées avec de la saumure (1 x 20 ml), séchées sur Na₂SO₄, filtrées et concentrées sous pression réduite. La matière brute a été purifiée par chromatographie flash (100 g SiO₂, éther de pétrole / acétate d'éthyle, 6:4) fournissant BR297 (1.25 g, 60 %, F: 106.2-107.3°C) ayant l'aspect d'un solide blanc.
**IR (ATR):** 1704.
**¹H NMR (CDCl₃):** 5.91 (1H, ddt, J= 5.7- 10.5- 17.1Hz); 5.25 (1H, dq, J= 1.5- 17.1Hz); 5.13 (1H, dq, J= 1.5- 10.5); 4.00 (2H, d, J= 5.7Hz, CH₂O); 3.25 (1H, tt, J= 4.7- 11.1 Hz, H₃); 2.51 (1H, t, J= 6Hz, H₁₇); 2.30- 0.55 (22H, m); 2.10 (3H, s, OAc); 0.79 (3H, s, Me); 0.59 (3H, s, Me).
**¹³C NMR (CDCl₃):** 209.8 (C=O); 135.6 (C=CH); 116.4 (C=CH₂); 77.3 (CH, C₃); 58.9 (CH₂O); 63.9 (CH); 56.7 (CH); 54.3 (CH); 44.8 (CH); 44.3 (C); 39.1 (CH₂); 37.0 (CH₂); 35.8 (C); 35.7 (CH); 35.5 (CH₂); 32.0 (CH₂); 31.5 (CH₃); 28.7 (CH₂); 28.2 (CH₂); 22.8 (CH₂); 21.2 (CH₂); 13.4 (CH₃); 12.3 (CH₃).

### Exemple 4: Synthèse du (3R,5S,10S,13S,17S)-17-acetyl-10,13-dimethylhexadecahydro-1H-cyclopenta[a]phenanthren-3-yl benzoate 3

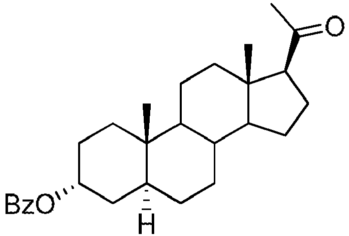

À une solution de composé 2 (1.95 g, 6.1 mmol) dans du THF (80 ml) ont été ajoutés de l'acide benzoique (1.12 g, 9.2 mmol), de la triphenylphosphine (2.40 g, 9.1 mmol) et du diisopropylazodicarboxylate (1.80 ml, 9.1 mmol). Le mélange réactionnel a été agité une nuit à température ambiante. De l'AcOEt (50 ml) a été ajouté et la phase organique a été lavée avec une solution de NaHCO₃ saturée (2 x 100 ml). Les phases aqueuses combinées ont été extraites avec de l'AcOEt (2 x 80 ml). Les phases organiques combinées ont été lavées avec de la saumure (1 x 100 ml), séchées sur Na₂SO₄, filtrées et concentrées sous pression réduite. La matière brute a été purifiée par chromatographie (250 g SiO₂, éther de pétrole / acétate d'éthyle, 9:1) fournissant le composé 3 (2.32 g, 90 %, F : 111-113.1°C) ayant l'aspect d'un solide blanc.
**IR (ATR):** 1703, 1449.
**¹H NMR (CDCl₃****):** 8.06 (2H, dd, J= 0.9- 8.1 Hz, H_{Ar}); 7.55 (1H, t, J= 7.5Hz, H_{Ar}); 7.46 (2H, t, J= 7.5Hz, H_{Ar}); 5.28 (1H, s, H₃); 2.52 (1H, t, J= 9Hz, H₁₇); 2.30-0.80 (22H, m); 2.12 (3H, s, H₂₁); 0.85 (3H, s, Me); 0.62 (3H, s, Me).
**¹³****C** NMR **(CDCl₃):** 209.7 (C=O, C₂₀); 165.9 (C=O, OBz); 132.7 (CH, C_{Ar}); 131.2 (C, C_{Ar}); 129.5 (2CH, C_{Ar}); 128.3 (2CH, C_{Ar}); 70.7 (CH, C₃); 63.9 (CH); 56.7 (CH); 54.2 (CH); 44.3 (C); 40.4 (CH); 39.1 (CH₂); 35.9 (C); 35.5 (CH); 33. (CH₂); 33.0 (CH₂); 31.9 (CH₂); 31.5 (CH₃); 28.3 (CH₂); 26.3 (CH₂); 24.4 (CH₂); 22.8 (CH₂); 20.8 (CH₂); 13.5 (CH₃); 11.4 (CH₃).

### Exemple 5: Synthèse de la 1-((3R,5S,10S,13S,17S)-3-hydroxy-10,13-dimethylhexadecahydro-1H-cyclopenta[a]phenanthren-17-yl)ethanone (ou allopregnanolone) 4

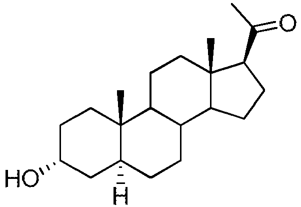

À une solution de composé 3 (390 mg, 0.88 mmol) dans du MeOH (10 ml) a été ajouté du KOH (298 mg, 5.32 mmol). Le mélange réactionnel a été chauffé sous reflux pendant 2 h 30. Après refroidissement à température ambiante, de l'eau (20 ml) a été ajoutée. La phase aqueuse a été extraite avec CH₂Cl₂ (3 x 20 ml). Les phases organiques combinées ont été séchées sur Na₂SO₄, filtrées et concentrées sous pression réduite. La matière brute a été purifiée par chromatographie (5 g SiO₂, éther de pétrole / acétate d'éthyle, 7:3) fournissant le composé 4 (223 mg, 80 %, F: 161.7-162.8°C) ayant l'aspect d'un solide blanc.
**IR (ATR)**: 3272, 1703.
**¹H NMR (CDCl₃):** 4.04 (1H, m, H₃); 2.58 (1H, t, J= 6Hz, H₁₇); 2.20- 1.90 (1H, m); 2.10 (3H, s, OAc); 1.97 (1H, dt, J= 2.7-11.7Hz); 1.80-0.65 (21H, m); 0.77 (3H, s, Me); 0.59 (3H, s, Me).
**¹³C NMR (CDCl₃):** 209.8 (C=O); 66.5 (CH, C₃); 63.8 (CH); 56.7 (CH); 54.2 (CH); 44.3 (C); 39.1 (CH); 36.1 (C); 35.8 (CH₂); 35.5 (CH); 32.2 (CH₂); 1.9 (CH₂); 31.5 (CH₃); 29.0 (CH₂); 28.5 (CH₂); 24.4 (CH₂); 22.8 (CH₂); 20.9 (CH₂); 13.5 (CH₃); 11.2 (CH₃).

### Exemple 6: Synthèse du (3R,5S,10S,13S,17S)-10,13-dimethyl-17-(2-methyl-1,3-dioxolan-2-yl)hexadecahydro-1H-cyclopenta[a]phenanthren-3-ol 5

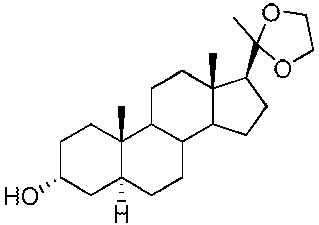

À une solution de composé 4 (221 mg, 0.69 mmol) dans le toluène (15 ml) ont été ajouté de l'éthylène glycol (0.60 ml, 10.92 mmol) et du pyridiniumparatoluenesulfonate (en quantité catalytique). Le mélange réactionnel a été chauffé sous reflux avec un dispositif Dean Stark pendant 3 h et le solvant a été évaporé sous pression réduite. Le résidu a été dissout dans du CH₂Cl₂ (30 ml) et lavé à l'eau (2 x 20 ml). Les phases aqueuses combinées ont été extraites avec CH₂Cl₂ (2 x 20 ml). Les phases organiques combinées ont été séchées sur Na₂SO₄, filtrées et concentrées sous pression réduite. La matière brute a été purifiée par chromatographie flash (10 g SiO₂, éther de pétrole / acétate d'éthyle, 9:1) fournissant le composé 5 (173 mg, 69 %) ayant l'aspect d'un solide amorphe blanc.
**IR (ATR):** 3281, 1705.
**¹H NMR (C₆D₆):** 3.78 (1H, m, H-3β); 3.65-3.45 (4H, m, dioxolane); 2.13 (1H, dt, J = 3.3- 12 Hz); 2.00-1.70 (3H, m); 1.70-0.60 (17H, m); 1.33 (3H, s, H-21); 0.96 (3H, s, Me); 0.70 (3H, s, Me).

### Exemple 7: Synthèse du 2-((3R,5S,10S,13S,17S)-3-(allyloxy)-10,13-dimethylhexadecahydro-1H-cyclopenta[a]phenanthren-17-yl)-2-methyl-1,3-dioxolane 6

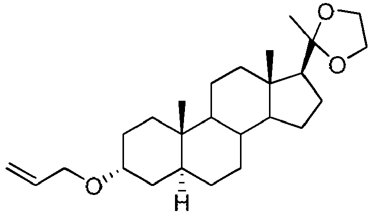

À une solution de composé 5 (472 mg, 1.32 mmol) dans du THF (60 ml) ont été ajoutés du NaH (327 mg, 13.2 mmol) et du bromure d'allyl (1.15 ml, 13.2 mmol). Le mélange réactionnel a été chauffé au reflux pendant 20 h. Après le refroidissement à température ambiante, de l'eau (40 ml) a été lentement ajoutée. La phase aqueuse a été extraite avec de l'AcOEt (3 x 40 ml). Les phases organiques combinées ont été lavées avec de la saumure (1 x 40 ml), séchées sur Na₂SO₄, filtrées et concentrées sous pression réduite. La matière brute a été purifiée par chromatographie flash (10 g SiO₂, éther de pétrole / acétate d'éthyle, 95:5) fournissant le composé 6 (440 mg, 83 %, F : 86.2-87.4°C) ayant l'aspect d'un solide blanc.
**IR (ATR):** 1447, 1369.
**¹H NMR (C₆D₆):** 6.02-5.86 (1H, m, C=CH); 5.32 (1H, dq, J = 1.5-17.1 Hz, C=CH₂); 5.06 (1H, dq, J = 1.2-10.2 Hz, C=CH₂); 3.85 (2H, d, J = 4.8 Hz, OCH2); 3.65-3.35 (5H, m, dioxolane and H₃); 2.14 (1H, dt, J = 3.1-12.2 Hz); 2.00-0.60 (23H, m); 1.32 (3H, s, H₂₁); 0.96 (3H, s, Me); 0.76 (3H, s, Me).
**¹³C NMR (C₆D₆):** 136.9 (CH, C=CH); 115.6 (CH₂, C=CH₂); 112.3 (C, C₂₀); 74.1 (CH, C₃); 69.4 (CH₂, OCH2); 65.5 (CH₂, dioxolane); 63.6 (CH₂, dioxolane); 59.3 (CH); 57.0 (CH); 54.8 (CH); 42.7 (C); 40.5 (CH); 40.2 (CH₂); 36.6 (C); 35.7 (CH); 34.0 (CH₂); 33.5 (CH₂); 32.7 (CH₂); 29.5 (CH₂); 26.6 (CH₂); 25.2 (CH₃); 24.4 (CH₂); 23.8 (CH₂); 21.4 (CH₂); 13.9 (CH₃); 12.0 (CH₃).

### Exemple 8: Synthèse de la 1-((3R,5S,10S,13S,17S)-3-(allyloxy)-10,13-dimethylhexadecahydro-1H-cyclopenta[a]phenanthren-17-yl)ethanone BR351

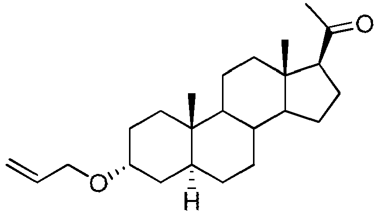

À une suspension de SiO₂ (5 g) dans du CH₂Cl₂ (5 ml) a été ajouté 20 gouttes d'une solution saturée d'acide oxalique. Le mélange a été agité 5 minutes et une solution de composé 6 (92 mg, 0.23 mmol) dans du CH₂Cl₂ (5 ml) a été ajoutée. Le mélange réactionnel a été agité 50 minutes à température ambiante. Le gel de silice a été filtré et lavé avec du CH₂Cl₂ (30 ml) et de l'AcOEt (30 ml). Les solvants ont été évaporés sous pression réduite. La matière brute a été purifiée par chromatographie (5 g SiO₂, éther de pétrole / acétate d'éthyle, 9:1) fournissant BR351 (77 mg, 93 %, F : 46.5-48.1 °C) ayant l'aspect d'un solide blanc.
**IR (ATR):** 1701, 1643.
**¹H NMR (C₆D₆):** 6.10-5.90 (1H, m, C=CH); 5.40-5.30 (1H, m, C=CH₂); 5.15-5.10 (1H, m, C=CH₂); 3.85-3.80 (2H, m, CH₂O); 3.44 (1H, t, J= 2.7Hz, H₃); 2.13 (1H, t, J= 6.3Hz, H₁₇); 1.90-0.50 (25H, m); 0.69 (3H, s, Me); 0.59 (3H, s, Me).
**¹³****C** NMR **(C₆D₆):** 209.0 (C=O); 136.1 (CH, C=CH); 114.9 (CH2, CH=CH₂); 73.3 (CH, C₃); 68.7 (CH₂, OCH2); 63.4 (CH); 65.3 (CH); 53.9 (CH); 39.4 (CH); 38.8 (CH₂); 35.2 (CH₃); 33.2 (CH₂); 32;7 (CH₂); 31.9 (CH₂); 28.6 (CH₂); 25.8 (CH₂); 24.2 (CH₂); 22.8 (CH₂); 20.7 (CH₂); 13.2 (CH₃); 11.2 (CH₃).

### Exemple 9: Synthèse du (3S,5S,10S,13S)-17-acetyl-10,13-dimethyl-12-oxo-2,3,4,5,6,7,8,9,10,11,12,13,14,15-tetradecahydro-1H-cyclopenta[a]phenanthren-3-yl acetate 7

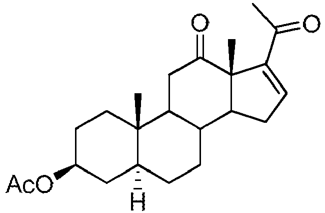

À une solution d'acétate d'hécogénine purifiée (5.02 g, 10.61 mmol) dans de l'Ac₂O (80 ml) ont été ajoutés du NH₄Cl (0.60 g, 11.21 mmol) et de la pyridine (1 ml, 12.36 mmol). Le mélange réactionnnel a été chauffé au reflux pendant 15 heures. La solution orange obtenue a été refroidie à 0 °C et a été ajoutée au goutte-à-goutte sur plus de 15 minutes une solution de CrO₃ (2.00 g, 20.00 mmol) dans l'eau (16 ml) et l'AcOH(6 ml).La solution noire a été agitée 1 h 30 à température ambiante puis diluée avec de l'eau (100 ml) et extraite avec du Et₂O (3 x 75 ml). Les phases organiques ont été lavées avec NaHCO₃ saturée (1 x 100 ml), avec de la saumure (1 x 100 ml), séchées sur Na₂SO₄, filtrées et concentrées sous pression réduite. Le résidu vert obtenu a été dissout dans de l'AcOH (50 ml) et a été chauffé 1 h au reflux. Le résidu brun obtenu après la concentration a été dissout dans du CH₂Cl₂ (50 ml) et lavé avec l'eau (2 x 50 ml), avec une solution de NaHCO₃ saturée (1 x 50 ml), séché sur Na₂SO₄, filtré et concentré sous pression réduite. Le brut a été purifié par chromatographie (150 g SiO₂, éther de pétrole / acétate d'éthyle, 8:2) fournissant le composé 7 (2.66 g, 67 %, F : 155.2-157.7°C) ayant l'aspect d'un solide blanc.
**IR (ATR):** 1721, 1710, 1672.
**¹H NMR (CDCl₃):** 6.60 (dd, 1H, *J* = 1.8- 3 Hz, H-16); 4.67 (m, 1H, H-3α); 2.58 (t, 1H, *J* = 13.2 Hz, H-11) ; 2.43 (ddd, 1H, *J* = 3.3- 6.9- 16.8 Hz, H-11); 2.32 (s, 3H, H-21); 2.30- 2.10 (m, 1H); 2.25 (dd, 1H, *J* = 5.4- 13.2 Hz); 2.05- 1.90 (1H, m); 2.02 (s, 3H, CH₃, Ac); 1.95 (dd, 1H, *J =* 3.4- 11.4 Hz); 1.88- 1.25 (m, 7H) 1.32 (s, 3H, CH₃); 1.25- 1.00 (m, 5H); 0.95 (s, 3H, CH₃).
**¹³C NMR (CDCl₃):** 209.7 (C=O, C-12); 196.3 (C=O, C-20); 170.5 (C=O, OAc); 150.6 (C, C-17); 142.4 (CH, C-16); 73.1 (CH, C-3); 61.1 (C); 56.7 (CH), 55.9 (CH); 44.6 (CH); 38.0 (CH₂); 36.3 (C); 36.1 (CH₂); 33.7 (CH₂); 33.4 (CH); 31.6 (CH₂); 31.4 (CH₂); 28.2 (CH₂); 27.4 (CH₃); 27.2 (CH₂); 21.4 (CH₃); 16.5 (CH₃); 11.8 (CH₃).
**HRMS (ESI) m/z:** C₂₃H₃₃O₄ [M+H⁺] calculé: 373.2373, trouvé : 373.2384.

### Exemple 10: Synthèse du (3S,5S,10S,13S,17S)-17-acetyl-10,13-dimethyl-12-oxohexadecahydro-1H-cyclopentafalphenanthren-3-yl acetate 8

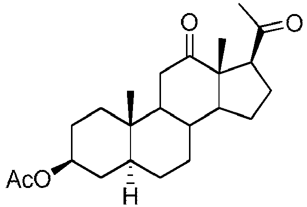

À une solution de composé 7 (2.78 g, 7.47 mmol) dans de l'EtOH (70 ml), a été ajouté du Pd/C à 10 % (0.33 g). Le mélange réactionnel a été ventillé avec de l'H2 (vide/H2) et a été agité vigoureusement sous H₂ jusqu'à l'absorption de 1.1 eq de H₂ (195 ml, 7.8 mmol). Le mélange réactionnel a été ventilé avec de l'Argon (vide/argon), le catalyseur a été filtré sur Celite et lavé avec de l'EtOH (50 ml), AcOEt (50 ml), CH₂Cl₂ (50 ml) puis concentré sous pression réduite. Le solide blanc obtenu a été purifié par chromatographie (100 g SiO₂, éther de pétrole/ acétate d'éthyle, 8:2) fournissant le composé 8 (2.257 g, 81 %, F : 189-192.6°C) ayant l'aspect d'un solide blanc.
**IR(ATR):** 1723, 1699.
**¹H NMR (CDCl₃):** 4.68 (tt, 1H, J = 4.9- 11.2 Hz, H-3α);3.30 (t, 1H, *J* = 9.3 Hz, H-17α); 2.48 (t, 1H, *J* = 13.2 Hz, H-11); 2.30- 2.05 (m, 2H); 2.26 (s, 3H, CH₃, Ac); 2.02 (s, 3H, CH₃, Ac); 1.90- 0.95 (15H, m); 0.94 (s, 3H, CH₃); 0.91 (s, 3H, CH₃).
**¹³C NMR (CDCl₃):** 212.7 (C=O, C-12); 209.1 (C=O, C-20); 169.0 (C=O; OAc); 72.5 (CH, C-3); 57.5 (C, C-13), 56.7 (CH, C-17); 55.5 (CH); 53.6 (CH); 43.8 (CH); 37.4 (CH₂); 35.7 (CH₂); 35.5 (C); 34.1 (CH₂); 33.1 (CH₂); 30.7 (CH); 30.6(CH₃); 27.5 (CH₂); 26.5 (CH₂); 23.4 (CH₂); 21.9 (CH₂), 20.7 (CH₃); 12.9 (CH₃); 11.2 (CH₃).
**HRMS (ESI) m/z:** C₂₃H₃₅O₄ [M+H⁺] calculé: 375.2530, trouvé: 375.2547.

### Exemple 11 : Synthèse de la (3S,5S,10S,13S,17S)-17-acetyl-3-hydroxy-10,13-dimethyltetradecahydro-1H-cyclopenta[a]phenanthren-12(2H)-one BR053

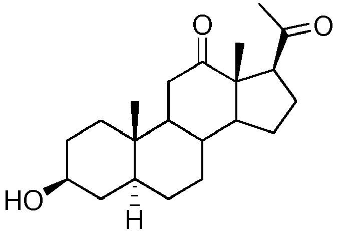

À une solution de composé 8 (384 mg, 1.03 mmol) dans un mélange de MeOH/THF (1/1, 10 ml) a été ajouté du K₂CO₃ (583 mg, 4.22 mmol). Le mélange réactionnel a été agité 5 h à température ambiante. De l'eau (10 ml) a été ajoutée. La phase aqueuse a été extraite avec du CH₂Cl₂ (2 x 20 ml) et avec de l'AcOEt (1 x 20 ml). Les phases organiques combinées ont été lavées avec de la saumure (1 x 20 ml), séchées sur Na₂SO₄, filtrées et concentrées sous pression réduite. La matière brute a été purifiée par chromatographie (10 g SiO₂, éther de pétrole / acétate d'éthyle, 6:4) fournissant le composé BR053 (317 mg, 93 %, F : 183.9-185.6°C) ayant l'aspect d'un solide blanc.
**IR (ATR):** 3389, 1712, 1697.
**¹H NMR (CDCl₃):** 3.60 (tt, 1H*, J =* 4.9-11.0 Hz, H-3α);3.31 (t, 1H*, J =* 9.3 Hz, H-17α); 2.49 (t, 1H*, J =* 13.2 Hz, H-11); 2.40-2.10 (m, 2H); 2.26 (s, 3H, CH₃, H-21); 1.95- 0.70 (m, 18H) ; 0.95 (s, 3H, CH₃); 0.90 (s, 3H, CH₃).
**¹³C NMR (CDCl₃):** 213.5 (C=O) ; 209.8 (C=O) ; 70.9 (CH, C-3) ; 58.2 (C, C-13) ; 57.5 (CH) ; 56.4 (CH) ; 54.3 (CH) ; 44.6 (CH) ; 38.1 (CH₂) ; 37.8 (CH₂); 36.6 (CH₂); 36.2 (C, C-10); 34.8 (CH₃); 31.4 (2CH₂); 28.3 (CH₂); 24.1 (CH₂); 22.6 (CH₂); 13.5 (CH₃); 11.9 (CH₃).

### Exemple 12: Synthèse du (3R,5S,10S,13S,17S)-17-acetyl-10,13-dimethyl-12-oxohexadecahydro-1H-cyclopenta[a]phenanthren-3-yl benzoate 9

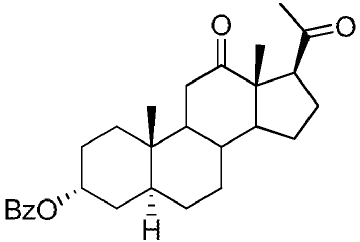

À une solution de composé 8 (2.11 g, 6.35 mmol) dans du THF (80 ml) ont été ajoutés de l'acide benzoique (1.17 g, 9.5 mmol), de la triphénylphosphine (2.52 g, 9.5 mmol) et du diisopropylazodicarboxylate (1.90 ml, 9.5 mmol). Le mélange réactionnel a été agité 15 h à température ambiante. De l'AcOEt (50 ml) a été ajouté et la phase organique a été lavée avec une solution de NaHCO₃ saturée (2 x 100 ml). Les phases aqueuses combinées ont été extraites avec de l'AcOEt (2 x 80 ml). Les phases organiques combinées ont été lavées avec de la saumure (1 x 100 ml), séché sur Na₂SO₄, filtré et concentré sous pression réduite. La matière brute a été purifiée par chromatographie sur colonne (250 g SiO₂, éther de pétrole / acétate d'éthyle, 9:1) fournissant le composé 9 (2.56 g, 92 %, F : 138.2-141.1°C) ayant l'aspect d'un solide blanc.
**IR (ATR):** 1702.
**¹H NMR (CDCl₃):** 8.02 (dd, H, *J* = 0.9-8.1 Hz, H-Ar); 7.54 (t, 1H*, J* = 7.2Hz, H-Ar); 7.44 (t, 2H, *J* = 7.2Hz, H-Ar); 5.29 (s, 1H, H-3β); 3.32 (t, 1H, *J* = 9.6 Hz, H-17α); 2.50 (t, 1H, *J* = 13.3 Hz, H-11); 2.40-2.10 (m, 1H) ; 2.30 (dd, 1H, *J* = 4.8-12 Hz, H-11) ; 2.26 (s, 3H, CH₃, Ac, H-21); 2.00-0.80 (m, 19H) ; 0.96 (s, 3H, CH₃) ; 0.93 (s, 3H, CH₃).
**¹³C NMR (CDCl₃) :** 213.6 (C=O); 209.7 (C=O); 165. (COOBz); 132.8 (CH, C-Ar); 130.9 (C, C-Ar); 129.5 (2CH, C-Ar), 128.4 (2CH, C-Ar); 70.1 (CH, C-3); 58.2 (C, C-13); 57.7 (CH); 56.5 (CH); 54.3 (CH); 40.3 (CH); 37.8 (CH₂); 36.6 (C, C-10); 34.8 (CH); 32.9 (CH₂); 32.7 (CH₂); 31.3 (CH₃); 28.0 (CH₂); 26.1 (CH₂); 24.1 (CH₂); 22.6 (CH₂); 13.5 (CH₃); 11.1 (CH₃).

### Exemple 13: Synthèse de la (3R,5S,10S,13S,17S)-17-acetyl-3-hydroxy-10,13-dimethyltetradecahydro-1H-cyclopenta[a]phenanthren-12(2H)-one BR338

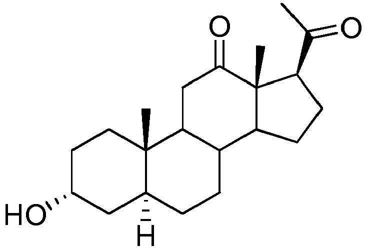

À une solution de composé 9 (2.44 g, 5.6 mmol) dans du MeOH (50 ml) a été ajouté du KOH (1.41 g, 25.2 mmol). Le mélange réactionnel a été chauffé au reflux pendant 1 h 30. Après refroidissement à température ambiante, de l'eau (40 ml) a été ajoutée. La phase aqueuse a été extraite avec du CH₂Cl₂ (3 x 40 ml). Les phases organiques combinées ont été séchées sur Na₂SO₄, filtrées et concentrées sous pression réduite. La matière brute a été purifiée par chromatographie (60 g SiO₂, éther de pétrole / acétate d'éthyle, 8:2) fournissant le composé BR338 (1.02 g, 55 %, F : 193.5-195.6°C) ayant l'aspect d'un solide blanc.
**IR (ATR):** 3490, 1698.
**¹H NMR (CDCl₃):** 4.05 (t, 1H*, J =* 2.7 Hz, H-3β) ; 3.30 (t, 1H*, J =* 8.7 Hz, H-17α) ; 2.44 (t, 1H, *J* = 13.5 Hz, H-11) ; 2.40-2.10 (m, 2H); 2.25 (s, 3H, CH₃, Ac, H-21) ; 1.90-1.10 (m, 18H) ; 0.93 (s, 3H, CH₃) ; 0.85 (s, 3H, CH₃).
**¹³C NMR (CDCl₃):** 213.7 (C=O) ; 209.9 (C=O) ; 66.1 (CH, C-3) ; 58.2 (C, C-13); 57.6 (CH); 56.4 (CH); 54.3 (CH); 38.9 (CH); 37.7 (CH₂); 36.8 (C, C-10); 34.8 (CH); 31.9 (CH₂); 31.4 (CH₂); 28.9 (CH₂); 28.9 (CH₂); 28.2 (CH₂); 24.0 (CH₂); 22.6 (CH₂); 13.6 (CH₃); 10.8 (CH₃).

### Exemple 14: Evaluation sur des cultures cellulaires in vitro des effets neuroprolifératif et neuroprotecteur des dérivés selon l'invention, ou conformes à l'invention

Le modèle cellulaire utilisé pour réaliser les expériences in vitro est une lignée de neuroblastomes humains dénommée lignée SH-SY5Y.

### A) Modes opératoires

### A.1. Culture cellulaire

Les neuroblastomes humains sont cultivés à 37°C en atmosphère saturée en humidité, sous 5% de CO₂ dans un milieu de culture constitué avec du DMEM additionné de 10% (v/v) de sérum de veau foetal, de 5% (v/v) de sérum de cheval, de 2 mM de glutamax et de 1% (v/v) du mélange pénicilline/streptomycine.

### A.2. Analyse microscopique de la morphologie des cellules SH-SY5Y

Pour s'assurer de la bonne qualité des neuroblastomes utilisés, 24h après la mise en culture, les cellules SH-SY5Y sont observées au microscope DMR équipé d'une caméra digitale assistée par un ordinateur PC pentium IV (Leica, microsystems, Wetzlan, Allemagne).

### A.3. Evaluation de la viabilité et de la densité des cellules: Détermination de l'effet des neurostéroïdes sur la prolifération cellulaire

La viabilité cellulaire et la densité (ou nombre de cellules SH-SY5Y/cm²) ont été déterminées grâce au *MTT reduction assay.* La technique de MTT est un test colorimétrique qui mesure la réduction du sel de tétrazolium (3-[4,5-diméthylthiazol-2-yl]-2,5-diphényl tetrazolium bromide) en formazan par la succinate déshydrogénase présente dans les mitochondries des cellules vivantes. Le formazan forme des cristaux de couleur bleue. Ainsi, après solubilisation des cristaux, la mesure de l'absorbance à 595 nm permet d'obtenir des valeurs d'absorbance directement proportionnelles aux nombres de cellules viables et à la densité cellulaire dans les boîtes de culture.

Les effets des dérivés BR053, BR338, BR297 et BR351 sur la prolifération cellulaire ont été évalués en comparant le nombre de cellules viables et la densité cellulaire dans les boîtes de cultures témoins, traitées uniquement avec le milieu de culture décrit dans le paragraphe A1 ci-dessus et les boîtes de cultures exposées au milieu de culture additionné du dérivé BR053, BR338, BR297 ou BR351. Les résultats sont regroupés dans le tableau 1 ci-dessous.

### A.4. Evaluation des effets neuroprotecteurs des dérivés selon l'invention ou conformes à l'invention contre la mort cellulaire induite par le stress oxydant

Les neuroblastomes humains ont été incubés avec du milieu de culture seul ou contenant le principal agent inducteur de stress oxydant, le peroxyde d'hydrogène (H₂O₂), à diverses concentrations afin de déterminer les concentrations d'H₂O₂ et les temps d'incubation induisant la mort d'un pourcentage significatif de cellules. Les propriétés neuroprotectrices des dérivés BR053, BR338, BR297 et BR351 ont été déterminées en prétraitant les cellules SH-SY5Y avec du milieu de culture seul ou contenant respectivement le dérivé BR053, BR338, BR297 ou BR351. Après le prétraitement, chacune de ces catégories de cellules a été incubée pour 24h ou 48h avec les solutions suivantes : (i) milieu de culture; (ii) milieu de culture contenant uniquement le dérivé BR053, BR338, BR297 ou BR351; (iii) milieu de culture contenant uniquement du H₂O₂ à la concentration de 1 mM qui provoque un fort pourcentage de mort cellulaire (iv) milieu de culture contenant à la fois du H₂O₂ (1 mM) et l'un des dérivés BR053, BR338, BR297 ou BR351.

10 µl d'une solution stock de tétrazolium, abrégé MTT, à 3,6 mM a été ajouté dans chaque puits et l'incubation est poursuivie à l'obscurité pendant 5h à 37°C. Après la lyse cellulaire, des mesures au spectrophotomètre à 595 nm ont été réalisées pour déterminer la survie et la densité cellulaire dans chacune des conditions mentionnées ci-dessus. Les résultats sont résumés dans le tableau 1 ci-dessous.

### A.5. Etude des effets des dérivés selon l'invention ou conformes à l'invention sur les récepteurs canaux GABA de type A (R-GABAa)

Ces expériences ont été réalisées pour vérifier si les dérivés BR053, BR338, BR297 et BR351 utilisent les mêmes mécanismes d'action que l'alloprégnanolone (3alpha, 5alpha-THP), notamment la modulation allostérique positive du R-GABAa, ou si ces dérivés (BR053, BR338, BR297 et BR351) présentent des différences dans leurs modalités d'action qui justifieraient le fait qu'ils exercent des activités spécifiques non évoquées par l'alloprégnanolone.

Les études ont été réalisées avec une approche électrophysiologique. L'activité des cellules a été enregistrée grâce à la technique du patch-clamp dans sa configuration dite cellule attachée. Les milieux utilisés pour les enregistrements avaient la composition suivante (en mM) : NaCl (132), KCI (2), CaCl₂ (1), MgCl₂ (2), HEPES (10) pour le milieu baignant les cellules et NaCl (132), KCI (2), CaCl₂ (1), MgCl₂ (2), TEA (15), HEPES (10) pour la pipette d'enregistrement, le pH était ajusté dans les deux cas à 7.4 avec du NaOH. Le GABA 10 µM a été ajouté dans la pipette d'enregistrement.

Après réalisation d'un « gigaseal » (Planar patch clamp) entre la membrane cellulaire et la pointe d'une pipette d'une résistance électrique comprise entre 4 et 7 MΩ, les courants ioniques on été mesurés grâce à un amplificateur de patch-clamp (Axopatch B200, Axon Instruments, CA). L'acquisition du signal (fréquence d'acquisition de 10 kHz) s'est effectuée à l'aide d'une carte interface (Digidata 1322A, Axon Instruments, CA) et du logiciel pClamp 8 (Axons Instruments, CA).

L'alloprégnanolone et les dérivés BR053, BR338, BR297 et BR351 ont été solubilisés dans de l'éthanol pur avant d'être dilués dans le milieu à la concentration souhaitée. Le facteur de dilution était au moins égal à 2x10⁴, ce qui fait que la concentration finale d'éthanol dans la solution d'enregistrement demeure inférieure à 1 µM (une concentration n'affectant pas l'activité du récepteur GABA_{A}).

Selon les expériences, l'alloprégnanolone ou les dérivés BR053, BR338, BR297 ou BR351, à la concentration de 0,25µM, étaient soit ajoutés dans la pipette d'enregistrement en même temps que le GABA, ou soit appliqués à la cellule par perfusion.

L'application se faisait par gravité à travers un système de perfusion multivoies constitué par des tubes en verre ayant chacun un diamètre interne de 1 mm. La voie sélectionnée était placée en face de la cellule enregistrée à environ 100 µm. Le changement de solution s'opérait par rotation calibrée des tubes grâce à un moteur pas à pas plaçant ainsi la nouvelle voie sélectionnée en face de la cellule. De manière synchrone à cette rotation s'effectuait aussi l'ouverture de l'électrovanne qui contrôle l'écoulement de la voie sélectionnée et la fermeture de la précédente.

### A.6. Mise en évidence des effets non-GABAergiques des dérivés selon l'invention ou conformes à l'invention

Ces expériences ont été réalisées dans les mêmes conditions d'enregistrement que les études électrophysiologiques décrites ci-dessus (A5) à l'exception du GABA qui n'a pas été rajouté dans la pipette d'enregistrement.

### B) Résultats

### B.1. Effet des dérivés selon l'invention sur la prolifération cellulaire

Les résultats récapitulés dans la colonne de gauche du Tableau 1 ci-dessous montrent clairement que le dérivé selon l'invention BR297 n'exerce aucun effet sur la prolifération des neuroblastomes humains. Ce dérivé BR297 est donc totalement dépourvu d'effet neuroprolifératif. En revanche, les autres dérivés stimulent la prolifération cellulaire avec une efficacité faible pour le BR053, importante pour le BR338 et très importante pour le BR351.

### B.2. Effet neuroprotecteur des dérivés selon l'invention contre la mort cellulaire induite par le stress oxydant

Comme le montrent les résultats regroupés dans la colonne de droite du Tableau 1 ci-dessous, tous les dérivés selon l'invention (BR053, BR338, BR297 et BR351) sont capables de protéger les neuroblastomes humains contre la mort cellulaire provoquée par une exposition au peroxyde d'hydrogène ou H₂O₂, un puissant inducteur de stress oxydant. Grâce à leur capacité de protéger les cellules nerveuses contre la mort cellulaire, ces dérivés peuvent donc être considérés comme des composés neuroprotecteurs. Toutefois, il convient de signaler que l'efficacité ou la puissance d'action neuroprotectrice est variable selon les dérivés. Ainsi, par ordre croissant d'efficacité neuroprotectrice, on peut citer le BR338, le BR297, le BR053 et le BR351 (à égalité avec le BR053).

**TABLEAU 1 :**

| **Effets des dérivés selon l'invention sur les cultures de neuroblastomes humains SH-SY55** | | |
|---|---|---|
| Composé | Effet sur la prolifération cellulaire (effet neuroprolifératif) | Effet neuroprotecteur contre la mort cellulaire induite par le stress oxydant |
| BR053 | + | +++ |
| BR338 | ++ | + |
| BR297 | - | ++ |
| BR351 | +++ | +++ |

Les pictogrammes « - », « + », « ++ », « +++ » signifient respectivement : sans effet, effet faible, effet important, effet très important.

### B.3. Effets des dérivés selon l'invention ou conformes à l'invention sur les récepteurs canaux GABA de type A (R-GABAa)

Comme le montrent les résultats regroupés dans le Tableau 2 ci-dessous, l'alloprégnanolone exerce un effet allostérique positif sur le canal R-GABAa. Cela signifie qu'en présence du GABA (contenu dans la pipette d'enregistrement) qui active son site sur le R-GABAa pour induire un courant ionique au niveau de la cellule enregistrée, l'alloprégnanolone, en agissant sur un autre site du R-GABAa, potentialise l'action du GABA en augmentant la perméabilité (amplitude) et la probabilité d'ouverture du canal. De manière intéressante, l'analyse comparative des effets de l'alloprégnanolone et des dérivés BR053, BR338, BR297 et BR351 sur le courant ionique induit par le GABA via le R-GABAa a révélé des similitudes d'action mais également des différences notables. En effet, les résultats montrent que, contrairement à l'alloprégnanolone, le dérivé BR053 induit une action allostérique négative sur le R-GABAa consistant à réduire l'amplitude et la probabilité d'ouverture du canal. Le BR338 mime parfaitement l'action de l'alloprégnanolone en exerçant comme ce neurostéroïde un effet allostérique positif résultant d'une augmentation de la perméabilité et de la probabilité d'ouverture du canal. Le BR297 ne mime que partiellement l'effet allostérique positif de l'alloprégnanolone en induisant une augmentation de la probabilité d'ouverture du canal R-GABAa sans augmenter sa perméabilité. Quant au dérivé BR351, il ne reproduit que de façon transitoire l'effet allostérique positif observé pour l'alloprégnanolone (augmentation de la perméabilité et de la probabilité d'ouverture du canal R-GABAa) et au bout d'une minute, le BR351 provoque une désensibilisation du canal R-GABAa.

**TABLEAU 2 :**

| **Effet sur le récepteur canal GABA de type A ou R-GABAa** | |
|---|---|
| Alloprég nanolone | Effet allostérique positif |
| BR053 | Effet allostérique négatif |
| BR338 | Effet allostérique positif |
| BR297 | Effet allostérique positif partiel |
| BR351 | Effet allostérique positif transitoire suivi d'une désensibilisation du canal |

### B.4. Effets non-GABAergiques des dérivés selon l'invention

Dans les conditions d'études électrophysiologiques où le GABA n'a pas été rajouté dans la pipette d'enregistrement, les dérivés BR053 et BR297 sont capables d'induire une activité cellulaire. Ces résultats démontrent que le BR053 et le BR297 exercent des effets non GABAergiques sur les cellules nerveuses.

### Exemple 15 : Evaluation in vivo des effets des dérivés selon l'invention sur la neuropathie douloureuse induite chez le rat par la chimiothérapie du cancer

### A) Modes opératoires

### A.1. Informations sur les animaux

Des rats mâles adultes Sprague-Dawley pesant entre 250 et 300 g ont été utilisés. Ils proviennent de la société commerciale Janvier (le Genest St Isle, France). Le soin et la manipulation des animaux ont été effectués en accord avec les directives du conseil de la communauté européenne (86/609/EC) et en adéquation avec les règles éthiques de *l'International Association for the Study of Pain.* Le protocole utilisé pour réaliser les travaux in vivo a fait l'objet d'un examen et d'une autorisation accordée à l'équipe du Professeur Mensah-Nyagan par le Département Alsacien du Guide de Santé Publique Vétérinaire pour les Soins et l'Utilisation des Animaux de Laboratoire (Agrément numéro 67-186). Les rats ont été hébergés dans des conditions standards de photopériode de 12 h jour/12 h nuit, avec de l'eau et de la nourriture à volonté. Les animaux ont passé une semaine d'acclimatation avant d'être intégrés dans les protocoles expérimentaux. Chaque animal était examiné quotidiennement afin de détecter d'éventuels signes de dégradation de son état de santé comme une perte dramatique du poids, une diminution de la prise de nourriture durant la chimiothérapie et le traitement pharmacologique. Une situation de souffrance n'a été observée chez aucun rat inclus dans la présente étude.

### A.2. Substances pharmacologiques et protocoles d'administration

La vincristine sulfate (VINC), achetée chez Sigma-Aldrich (St Louis, MO, USA), a été dissoute dans une solution physiologique saline (NaCl 0,9%) à 0,1 mg/ml et stockée à 4°C. La solution saline (NaCl 0,9%) est utilisée comme véhicule. La VINC est injectée quotidiennement par voie intrapéritonéale (i.p), en 2 cycles de 5 jours séparés par 2 jours de pause, à une concentration de 0,1 mg/kg/jour. Les animaux témoins ont été injectés en i.p (1 ml/kg) avec le véhicule selon un protocole similaire.

L'alloprégnanolone et les dérivés selon l'invention (BR053 et BR297) ont été dilués dans une solution d'hydroxypropylcellulose ou HPC (0,3%) utilisée comme véhicule. Le traitement curatif avec l'alloprégnanolone, le BR053 ou le BR297 a commencé 3 jours après les cycles de vincristine. Chacun de ces composés (l'alloprégnanolone, le BR053 ou le BR297) a été administré par voie intrapéritonéale à la dose de 4 mg/kg à raison d'une injection tous les 2 jours. Ainsi, sur la durée totale des expériences in vivo (14 jours), chaque animal a reçu selon son groupe d'appartenance 7 injections de véhicule, d'alloprégnanolone, de BR053 ou de BR297 à la dose de 4 mg/kg. Les animaux ont été sacrifiés 24h après le dernier test comportemental (28^{ème} jour) pour effectuer le prélèvement des tissus qui serviront à des études ultérieures d'histologie et d'électrophysiologie.

### A.3. Evaluation du seuil de sensibilité nociceptive mécanique avec le test des filaments de Von Frey

Le seuil de sensibilité nociceptive mécanique a été évalué chez des rats placés individuellement dans des boîtes de Plexiglas (30 x 30 x 25 cm) montées sur un support grillagé surélevé permettant ainsi d'accéder à la surface ou voûte plantaire des pattes postérieures. La présence d'une allodynie (réponse provoquée par un stimulus normalement non nocif) ou d'une hyperalgie mécanique (réponse exagérée à la suite d'une stimulation normalement douloureuse) a été mesurée en utilisant une série de filaments de von Frey calibrés (1, 2, 4, 6, 8, 10, 15, 26, 100, 180 et 300 g; Stoelting, Wood Dale, IL, USA) qui ont été appliqués sur la surface plantaire de la patte postérieure de l'animal avec une force croissante jusqu'à ce que le filament se torde. Le filament a été appliqué pendant une période de 1 à 2 sec et la procédure est répétée 5 fois avec un intervalle de 4-5 sec. Le seuil de retrait de la patte a été calculé en faisant la moyenne des 10 stimuli répétés (en g) qui induisent un réflexe de retrait de la patte. Seuls les retraits vigoureux et rapides de la patte ont été considérés comme positifs. Dans les expériences, les animaux naïfs n'ont jamais répondu au filament de 4g et répondent 25-30% des fois au filament de 15g. L'observation de réponses pour le filament de 4g après l'administration d'un traitement a révèlé une allodynie mécanique et l'augmentation des réponses au filament de 15g indique une hyperalgie mécanique.

### A.4. Evaluation de l'allodynie thermique au froid par le test de l'acétone

Le seuil de sensibilité nociceptive thermique au froid a été mesuré chez des rats sont placés individuellement dans des boîtes de Plexiglas (30 x 30 x 25 cm) montées sur un support grillagé surélevé afin de permettre l'accès à la voûte plantaire de l'animal. 50 µL d'acétone ont été déposés sur la patte de l'animal et les rats ont été observés pendant 20 sec. Six mesures (3 par patte) sont effectuées avec une latence de 5 minutes entre chaque stimulation. La latence moyenne de retrait des pattes (en secondes) a été calculée en moyennant 6 mesures à chaque point temporel étudié. Des animaux naïfs ne répondent pas à l'application d'acétone. Une diminution significative de la latence de retrait des pattes indique une allodynie thermique au froid.

### B) Résultats

Le traitement chimiothérapique à la vincristine induit une nette diminution du seuil de sensibilité nociceptive mécanique. Avant le traitement à la vincristine, le filament de Von Frey qui provoque plus de 70% de réponses (retrait de la patte) est de 80 g. Après la chimiothérapie à la vincristine, un filament de 20 g suffit pour produire plus de 70 % de réponses. De plus, les animaux traités à la vincristine développent une nette allodynie mécanique car le filament de 4 g qui n'induisait aucune réponse avant le traitement, provoque plus de 30 % de réponses après le traitement à la vincristine. Une hyperalgie mécanique est également apparue chez les animaux traités à la vincristine car le filament de 15 g qui n'entraînant que 30 % de réponses avant l'administration de la vincristine provoque pratiquement 70% de réponses après la chimiothérapie à la vincristine.

Par ailleurs, le test à l'acétone démontre une diminution drastique de la latence de retrait de la patte (qui chute de 20 à 11 secondes) après le traitement à la vincristine, ce qui met en évidence une allodynie thermique au froid chez les animaux traités.

De façon intéressante, chacun des neurostéroïdes, l'alloprégnanolone ou les dérivés selon l'invention, le BR053 ou le BR297, utilisé à la dose de 4 mg/kg de poids corporel, supprime avantageusement les symptômes d'allodynie mécanique, d'hyperalgie mécanique et d'allodynie thermique. En effet, l'administration pendant 2 semaines (une injection tous les 2 jours) d'alloprégnanolone, de BR053 ou de BR297 à 4mg/kg de poids corporel, restaure avantageusement chez les rats victimes d'une neuropathie douloureuse induite par la vincristine, des seuils normaux ou physiologiques des sensibilités nociceptives thermique et mécanique. Ce rétablissement des seuils normaux des sensibilités nociceptives thermique et mécanique témoigne du fait que l'alloprégnanolone ou les dérivés selon l'invention, le BR053 et le BR297, ont entrainé une suppression complète de la douleur neuropathique évoquée par la chimiothérapie à la vincristine.

Chez les animaux sains n'ayant pas été traités à la vincristine, l'administration pendant 2 semaines d'alloprégnanolone (4 mg/kg de poids corporel tous les 2 jours) induit un important effet anti-nociceptif caractérisé par une nette augmentation du seuil de sensibilité nociceptive mécanique qui passe de 80 g à 200 g. Contrairement à l'alloprégnanolone, l'administration pendant 2 semaines des dérivés selon l'invention, le BR053 ou le BR 297 (4 mg/kg de poids corporel tous les 2 jours), ne provoque aucun effet anti-nociceptif chez les animaux sains n'ayant pas été traités à la vincristine.

### C) Conclusions

Les dérivés selon l'invention tels que définis ci-dessus ou le dérivé de formule BR053, définie ci-dessus sont particulièrement utiles, chez le sujet neuropathique, comme composés permettant de supprimer définitivement la douleur grâce à leur capacité de réparer les dommages tissulaires et fonctionnels responsables de la neuropathie. Des expériences réalisées chez des rats souffrant d'une neuropathie douloureuse induite par la vincristine démontrent qu'un traitement de deux semaines avec le composé BR297 selon l'invention ou avec le dérivé BR053 et utilisés à raison d'une injection intrapéritonéale tous les 2 jours à la dose de 4 mg par Kg de poids corporel, permet de réparer les déficits fonctionnels évoqués par la vincristine et de supprimer définitivement la douleur neuropathique. Des données expérimentales supplémentaires révèlent que l'analgésie définitive produite par le BR297 ou le BR053 chez l'animal neuropathique n'est pas la conséquence d'un simple blocage de la transmission des messages nocifs (effet anti-nociceptif) ou d'une sédation induite par le BR297 ou le BR053. En effet, l'analyse comparative de l'action de l'alloprégnanolone, du BR297 et du BR053 sur la transmission nociceptive chez le rat sain montre qu'à la dose de 4 mg par Kg de poids corporel l'alloprégnanolone exerce un effet anti-nociceptif lié à son pouvoir sédatif alors qu'à la même dose le BR297 et le BR053 n'induisent pas d'action anti-nociceptive significative. En conséquence, la seule possibilité dont disposent les composés BR297 et BR053 pour induire l'analgésie complète consistant à supprimer définitivement la douleur neuropathique chez l'animal pathologique reste la réparation préalable des dégâts tissulaires et fonctionnels responsables de la neuropathie.

En outre, de façon particulièrement intéressante, les dérivés selon l'invention peuvent se distinguer entre eux par leurs effets sur la prolifération cellulaire.

Ainsi, le BR297, qui est un agent neuroprotecteur efficace n'exerçant aucun effet sur la prolifération cellulaire, suscite un véritable espoir pour le traitement et l'éradication définitive des neuropathies douloureuses induites par la chimiothérapie ou la radiothérapie du cancer. En effet, le BR297 peut donc être avantageusement prescrit chez les patients souffrant de neuropathies provoquées par le traitement cancéreux, que le cancer soit hormono-dépendant ou non.

En revanche, des composés tel que par exemple le BR053 (ayant un faible effet neuro-prolifératif), le BR338 et le BR351 (inducteurs d'une forte prolifération neuronale) peuvent s'avérer contre-indiqués chez le patient cancéreux, en particulier dans les cas de cancers hormono-dépendants. Néanmoins, ces neurostéroïdes (BR053, BR338 et BR351) présentent avantageusement un fort potentiel thérapeutique pour le traitement des pathologies neurodégénératives chez des sujets n'ayant pas de surcroît un cancer. En particulier, le BR351 qui possède une importante activité neuroproliférative et une efficacité élevée dans la neuroprotection, suscite un véritable espoir pour le traitement des maladies neurodégénératives dont l'éradication nécessite une réactivation de la neurogenèse dans le cerveau adulte pour compenser les pertes neuronales dans les territoires corticaux lésés et une protection préventive contre la dégénérescence des structures cérébrales encore intactes chez le patient. La stratégie thérapeutique basée sur l'utilisation du BR351 peut être bénéfique pour des millions de patients atteints de diverses pathologies neurodégénératives dans le monde.

## Revendications

1. Dérivé de l'alloprégnanolone ou 1-((3R,5S,10S,13S,17S)-3-hydroxy-10,13-dimethylhexadecahydro-1H-cyclopenta[a]phenanthren-17-yl)ethanone ou de l'épi-alloprégnanolone ou 1-((3S,5S,10S,13S,17S)-3-hydroxy-10,13-dimethylhexadecahydro-1H cyclopenta[a]phenanthren-17-yl)ethanone, de formule (I) suivante : 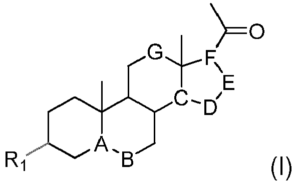 dans laquelle
• **R¹** est un groupement choisi parmi les groupements 3-alpha ou 3-beta hydroxy, les groupements 3-alpha ou 3-beta O-allyl, les groupements 3-alpha ou 3-beta O-propargyl, les groupements 3-alpha ou 3-beta O-glycol, les groupements 3-alpha ou 3-beta O-PEG, les groupements 3-alpha ou 3-beta O-glycol-allyl et les groupements 3-alpha ou 3-beta O-PEG-allyl,
• **A** est un atome de carbone substitué par un atome choisi parmi le 5-H alpha et le 5-H beta et **B** est un groupement méthylène ; ou
**A** et **B** sont des atomes de carbone formant une double liaison 5-6 ;
• **C** est un atome de carbone substitué par un atome choisi parmi le 14-H alpha, le 14-H beta, le 14-alpha OH et le 14-beta OH et **D** est un groupement méthylène ; ou
**C** et **D** sont des atomes de carbone formant une double liaison 14-15 ;
• **F** est un atome de carbone substitué par un atome choisi parmi le 17-H alpha et le 17-H beta et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
**F** est un atome de carbone substitué par un groupement choisi parmi le 17-alkyl-alpha, le 17-alkyl-beta, le 17-OR²-alpha et le 17-OR²-beta, avec R² un groupement choisi parmi le allyl, propargyl, glycol, PEG, glycol-allyl, PEG-allyl, et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
**E** et **F** sont impliqués dans un cycle époxy ou un cyclopropyle et sont choisis parmi le 16, 17-époxy-alpha, le 16, 17-époxy-beta, le 16,17-méthylène-alpha et le 16, 17-méthylène-beta ; ou
**E** et **F** sont des atomes de carbone formant une double liaison 16-17 ;
• **G** est un carbonyle, un méthylène ou un atome de carbone substitué par un groupement 12-OR3 alpha ou 12-OR3 beta avec R3 un atome d'H ou un groupement choisi parmi les groupements acétyle, alkyle et aryle,
R³ pouvant être choisi parmi les alkyl_{C1-C6}, le benzyle, le p-méthoxybenzyle, le benzoyle, le tigloyle, l'angeloyle, le 2,2,2-trichloroéthoxycarbonyle, le o-aminobenzoyle, le nicotinoyle, le 2-méthylbutyryle, l'isovaleryle, le cinnamoyle, le coumaroyle, le o-hydroxybenzoyle et l'anthraniloyle,
à l'exclusion de (i) la molécule de formule (BR053) suivante : 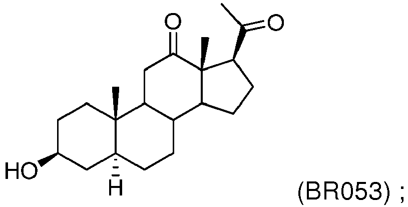 et
de (ii) la molécule de formule (BR338) suivante :

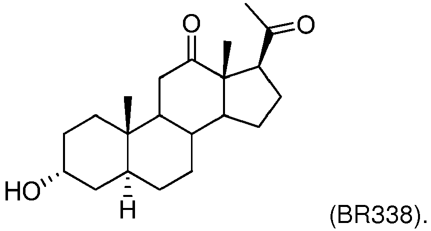

2. Dérivé selon la revendication 1 où
• **R¹** est un groupement choisi parmi les groupements 3-alpha ou 3-beta O-allyl, les groupements 3-alpha ou 3-beta O-propargyl, les groupements 3-alpha ou 3-beta O-glycol, les groupements 3-alpha ou 3-beta O-PEG, les groupements 3-alpha ou 3-beta O-glycol-allyl et les groupements 3-alpha ou 3-beta O-PEG-allyl,
• **A** est un atome de carbone substitué par un atome choisi parmi le 5-H alpha et le 5-H beta et **B** est un groupement méthylène ; ou
**A** et **B** sont des atomes de carbone formant une double liaison 5-6 ;
• **C** est un atome de carbone substitué par un atome choisi parmi le 14-H alpha, le 14-H beta, le 14-alpha OH et le 14-beta OH et **D** est un groupement méthylène ; ou
**C** et **D** sont des atomes de carbone formant une double liaison 14-15 ;
• **F** est un atome de carbone substitué par un atome choisi parmi le 17-H alpha et le 17-H beta et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
**F** est un atome de carbone substitué par un groupement choisi parmi le 17-alkyl-alpha, le 17-alkyl-beta, le 17-OR²-alpha et le 17-OR²-beta, avec R² un groupement choisi parmi le allyl, propargyl, glycol, PEG, glycol-allyl, PEG-allyl, et **E** est un groupement méthylène ou un atome de carbone substitué par un groupement choisi parmi le 16-alkyl-alpha, le 16-alkyl-beta, le 16-OR²-alpha et le 16-OR²-beta, avec R² un groupement choisi parmi le allyl, propargyl, glycol, PEG, glycol-allyl, PEG-allyl, ou
**E** et **F** sont impliqués dans un cycle époxy ou un cyclopropyle et sont choisis parmi le 16, 17-époxy-alpha, le 16, 17-époxy-beta, le 16,17-méthylène-alpha et le 16, 17-méthylène-beta ; ou
**E** et **F** sont des atomes de carbone formant une double liaison 16-17 ;
• **G** est un carbonyle, un méthylène ou un atome de carbone substitué par un groupement 12-OR3 alpha ou 12-OR3 beta avec R3 un atome d'H ou un groupement choisi parmi les groupements acétyle, alkyle et aryle,
R³ pouvant être choisi parmi les alkyl_{C1-C6}, le benzyle, le p-méthoxybenzyle, le benzoyle, le tigloyle, l'angeloyle, le 2,2,2-trichloroéthoxycarbonyle, le o-aminobenzoyle, le nicotinoyle, le 2-méthylbutyryle, l'isovaleryle, le cinnamoyle, le coumaroyle, le o-hydroxybenzoyle et l'anthraniloyle.

3. Dérivé selon la revendication 2 **caractérisé en ce que** c'est un dérivé de formule (I) dans laquelle **R¹** est un groupement choisi parmi les groupements 3-beta O-allyl, 3-beta O-propargyl, 3-beta O-glycol, 3-beta O-PEG, 3-beta O-glycol-allyl et 3-beta O-PEG-allyl et /ou **G** est un méthylène.

4. Dérivé selon l'une quelconque des revendications 1 à 3 de formule (BR297) suivante :

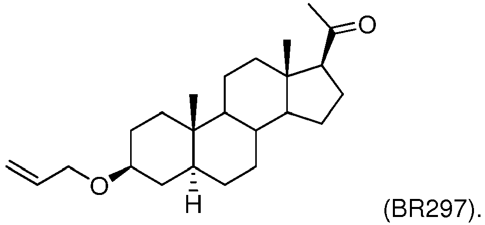

5. Dérivé selon l'une quelconque des revendications 1 à 3 de formule (BR351) suivante :

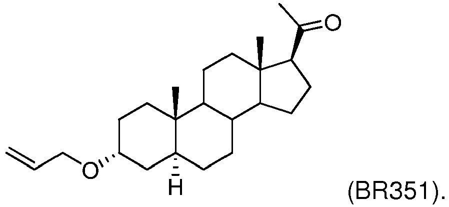

6. Dérivé selon l'une des revendications 1 à 5 pour son utilisation comme médicament.

7. Dérivé selon l'une des revendications 1 à 5 ou dérivé de formule (BR338) suivante : 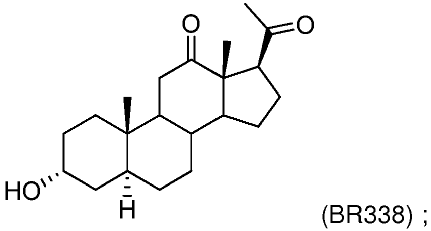 ou de formule (BR053) suivante : 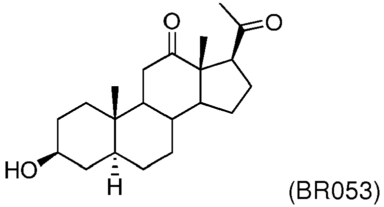 pour son utilisation comme agent neuroprotecteur et/ou comme agent stimulant la prolifération des cellules nerveuses.

8. Dérivé selon la revendication 7 pour le traitement d'une neuropathologie.

9. Dérivé pour le traitement selon la revendication 8 dans laquelle la neuropathologie est choisie parmi les neuropathies périphérique et centrale, la douleur chronique, les maladies neurodégénératives et les déficits sensorimoteurs induits par la neuroinflammation.

10. Dérivé pour le traitement selon la revendication 9 dans laquelle la neuropathologie est choisie parmi les neuropathies périphérique et centrale.

11. Dérivé pour le traitement selon la revendication 8 dans laquelle la neuropathologie est liée à une allodynie ou à une hyperalgésie.

12. Dérivé pour le traitement selon l'une des revendications 9 ou 10 dans laquelle la neuropathie est induite par un traitement anticancéreux, en particulier un traitement chimiothérapique mettant en oeuvre un antinéoplasique avantageusement choisi parmi les poisons du fuseau, les agents intercalants, les inhibiteurs des DNA topoisomérases, les inhibiteurs de la tyrosine kinase, les inhibiteurs des mTOR, les anticorps monoclonaux anti-angiogènes, les inhibiteurs de la synthèse et de la réplication de l'ADN et les inhibiteurs de la polymérisation ou de la dépolymérisation des microtubules, encore plus avantageusement choisi parmi la vincristine, l'oxaliplatine, le docétaxel, le paclitaxel et autres dérivés du Taxol®.

13. Dérivé selon l'une quelconque des revendications 1 à 5 ou dérivé de formule (BR338) suivante : 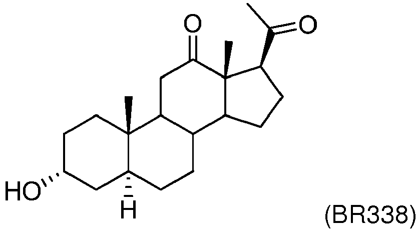 ou de formule (BR053) suivante : 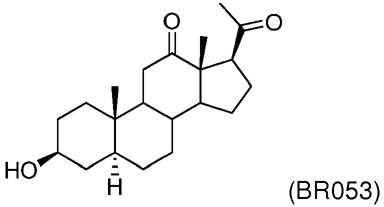 pour son utilisation comme neuroprotecteur, en particulier comme neuroréparateur.

14. Dérivé selon l'une quelconque des revendications 1, 2, 3 ou 5 ou dérivé de formule (BR338) suivante : 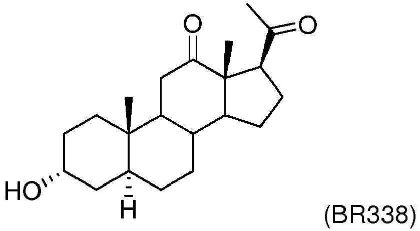 ou de formule (BR053) suivante : 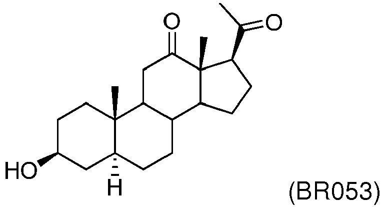 à l'exclusion du dérivé de formule (BR297) suivante : 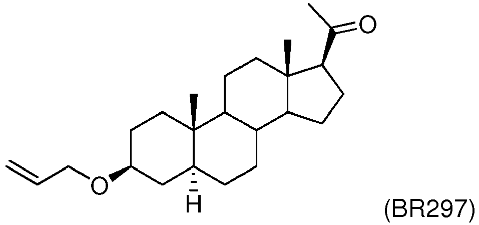 pour son utilisation comme agent stimulant la prolifération des cellules nerveuses.

15. Dérivé selon la revendication 4 ou dérivé de formule (BR053) suivante : 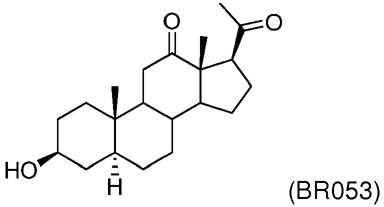 pour son utilisation à une dose de 4 mg dudit dérivé par kg de poids corporel, tous les 2 jours, chez un patient atteint d'une neuropathologie telle que définie selon l'une des revendications 9 à 10.

16. Dérivé selon l'une des revendications 1 à 5 ou dérivé de formule (BR338) suivante : 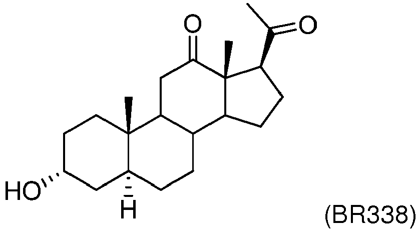 ou de formule (BR053) suivante : 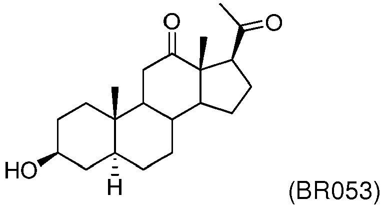 pour prévenir la mort cellulaire des neuroblastomes induite par le stress oxydant.

## Patentansprüche

1. Ein Derivat von Allopregnanolon oder 1-((3R,5S,10S,13S,17S)-3-Hydroxy-10,13-dimethylhexadecahydro-1H-cyclopenta[a]phenanthren-17-yl)ethanon oder von Epiallopregnanolon oder 1-((3S,5S,10S,13S,17S)-3-Hydroxy-10,13-dimethylhexadecahydro-1H cyclopenta[a]phenanthren-17-yl)ethanon entsprechend der folgenden Formel (I): 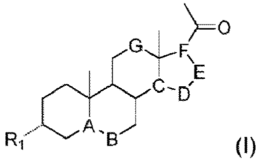 worin
• **R¹** eine Gruppe ist, ausgewählt aus 3-alpha oder 3-beta Hydroxygruppen, 3-alpha oder 3-beta O-Allylgruppen, 3-alpha oder 3-beta O-Propargylgruppen, 3-alpha oder 3-beta O-Glycolgruppen, 3-alpha oder 3-beta O-PEG-Gruppen, 3-alpha oder 3-beta O-Glycol-Allylgruppen und 3-alpha oder 3-beta O-PEG-Allylgruppen,
• **A** ein Kohlenstoffatom ist, das durch ein aus 5-H alpha und 5-H beta ausgewähltes Atom substituiert ist, und **B** eine Methylengruppe ist; oder
**A** und **B** Kohlenstoffatome sind, die eine 5,6-Doppelbindung bilden;
• **C** ein Kohlenstoffatom ist, das durch ein aus 14-H alpha, 14-H beta, 14-alpha OH und 14-beta OH ausgewähltes Atom substituiert ist, und **D** eine Methylengruppe ist; oder
**C** und **D** Kohlenstoffatome sind, die eine 14,15-Doppelbindung bilden;
• **F ein** Kohlenstoffatom ist, das durch ein aus 17-H alpha und 17-H beta gewähltes Atom substituiert ist, und **E** eine Methylengruppe oder ein Kohlenstoffatom ist, das durch eine Gruppe substituiert ist, ausgewählt aus 16-Alkyl-alpha, 16-Alkyl-beta, 16-OR²-alpha und 16-OR²-beta, mit R² einer aus Allyl, Propargyl, Glycol, PEG, Glycol-Allyl, PEG-Allyl ausgewählten Gruppe, oder
• **F** ein Kohlenstoffatom ist, das durch eine Gruppe substituiert ist, ausgewählt aus 17-Alkyl-alpha, 17-Alkyl-beta, 17-OR²-alpha and 17-OR²-beta, mit R² einer aus Allyl, Propargyl, Glycol, PEG, Glycol-Allyl, PEG-Allyl ausgewählten Gruppe, und **E** eine Methylengruppe oder ein Kohlenstoffatom ist, das durch eine Gruppe substituiert ist, ausgewählt aus 16-Alkyl-alpha, 16-Alkyl-beta, 16-OR²-alpha und 16-OR²-beta, mit R² einer aus Allyl, Propargyl, Glycol, PEG, Glycol-Allyl, PEG-Allyl ausgewählten Gruppe, oder
**E** und **F** zu einem Epoxy- oder einem Cyclopropylring gehören und ausgewählt sind aus 16,17-Epoxy-alpha, 16,17-Epoxy-beta, 16,17-Methylen-alpha und 16,17-Methylen-beta; oder
**E** und **F** Kohlenstoffatome sind, die eine 16,17-Doppelbindung bilden;
• **G** ein Carbonyl-, ein Methylen- oder ein Kohlenstoffatom ist, das durch eine 12-OR³-alpha- oder 12-OR³-beta-Gruppe substituiert ist, wobei R³ ein H-Atom oder eine Gruppe ausgewählt aus Acetyl-, Alkyl- und Arylgruppen ist,
R³ aus C₁-C₆-Alkyl, Benzyl, p-Methoxybenzyl, Benzoyl, Tigloyl, Angeloyl, 2,2,2-Trichloroethoxycarbonyl, o-Aminobenzoyl, Nicotinoyl, 2-Methylbutyryl, Isovaleryl, Cinnamoyl, Cumaroyl, o-Hydroxybenzoyl und Anthraniloyl ausgewählt werden kann,
unter Ausschluss (i) des Moleküls mit der folgenden Formel (BR053): 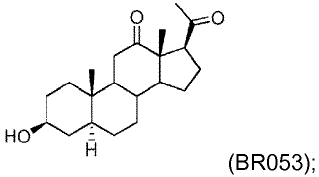 und
(ii) des Moleküls mit der folgenden Formel (BR338)

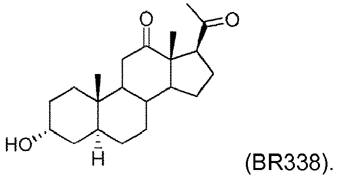

2. Derivat nach Anspruch 1, worin
• **R¹** eine Gruppe ist, ausgewählt aus 3-alpha oder 3-beta Allylgruppen, 3-alpha oder 3-beta O-Propargylgruppen, 3-alpha oder 3-beta O-Glycolgruppen, 3-alpha oder 3-beta O-PEG-Gruppen, 3-alpha oder 3-beta O-Glycol-Allylgruppen und 3-alpha oder 3-beta O-PEG-Allylgruppen,
• **A** ein Kohlenstoffatom ist, das durch ein aus 5-H alpha und 5-H beta gewähltes Atom substituiert ist, und **B** eine Methylengruppe ist; oder
**A** und **B** Kohlenstoffatome sind, die eine 5,6-Doppelbindung bilden;
• **C** ein Kohlenstoffatom ist, das durch ein aus 14-H alpha, 14-H beta, einer 14-alpha OH-Gruppe und 14-beta OH-Gruppe ausgewähltes Atom substituiert ist, und D eine Methylengruppe ist; oder
**C** und **D** Kohlenstoffatome sind, die eine 14,15-Doppelbindung bilden;
• **F** ein Kohlenstoffatom ist, das durch ein aus 17-H alpha und 17-H beta ausgewähltes Atom substituiert ist, und **E** eine Methylengruppe oder ein Kohlenstoffatom ist, das durch eine Gruppe substituiert ist, ausgewählt aus 16-Alkyl-alpha, 16-Alkyl-beta, 16-OR²-alpha und 16-OR²-beta, mit R² einer aus Allyl, Propargyl, Glycol, PEG, Glycol-Allyl, PEG-Allyl ausgewählten Gruppe, oder
• **F** ein Kohlenstoffatom ist, das durch eine Gruppe substituiert ist, ausgewählt aus 17-Alkyl-alpha, 17-Alkyl-beta, 17-OR²-alpha und 17-OR²-beta, mit R² einer aus Allyl, Propargyl, Glycol, PEG, Glycol-Allyl, PEG-Allyl ausgewählten Gruppe, und **E** eine Methylengruppe oder ein Kohlenstoffatom ist, das durch eine Gruppe substituiert ist, ausgewählt aus 16-Alkyl-alpha, 16-Alkyl-beta, 16-OR²-alpha und 16-OR²-beta, mit R² einer aus Allyl, Propargyl, Glycol, PEG, Glycol-Allyl, PEG-Allyl ausgewählten Gruppe, oder
**E** und **F** zu einem Epoxy- oder einem Cyclopropylring gehören und ausgewählt sind aus 16,17-Epoxy-alpha, 16,17-Epoxy-beta, 16,17-Methylen-alpha und 16,17-Methylen-beta; oder
**E** und **F** Kohlenstoffatome sind, die eine 16,17-Doppelbindung bilden;
• **G** ein Carbonyl-, ein Methylen- oder ein Kohlenstoffatom ist, das durch eine 12-OR³-alpha- oder 12-OR³-beta-Gruppe substituiert ist, wobei R³ ein H-Atom oder eine Gruppe ausgewählt aus Acetyl, Alkyl und Arylgruppen ist,
R³ aus C₁-C₆-Alkyl, Benzyl, p-Methoxybenzyl, Benzoyl, Tigloyl, Angeloyl, 2,2,2-Trichloroethoxycarbonyl, o-Aminobenzoyl, Nicotinoyl, 2-Methylbutyryl, Isovaleryl, Cinnamoyl, Cumaroyl, o-Hydroxybenzoyl und Anthraniloyl ausgewählt werden kann.

3. Derivat nach Anspruch 2, **dadurch gekennzeichnet, dass** es ein Derivat der Formel (I) ist, worin **R¹** eine aus 3-beta O-Allyl-, 3-beta O-Propargyl-, 3-beta O-Glycol-, 3-beta O-PEG-, 3-beta O-Glycol-Allyl- und 3-beta O-PEG-Allyl-Gruppen ausgewählte Gruppe ist und/oder **G** ein Methylen ist.

4. Derivat nach einem der Ansprüche 1 bis 3 mit der folgenden Formel (BR297):

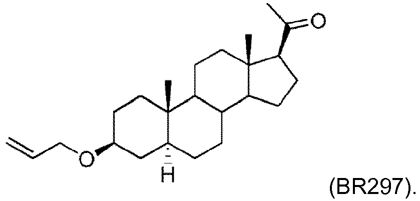

5. Derivat nach einem der Ansprüche 1 bis 3 mit der folgenden Formel (BR351):

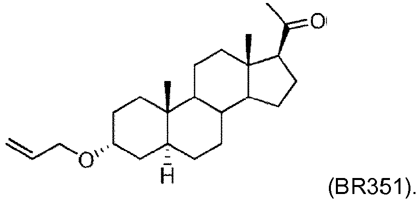

6. Derivat nach einem der Ansprüche 1 bis 5 zur Verwendung als Medikament.

7. Derivat nach einem der Ansprüche 1 bis 5 oder Derivat mit folgender Formel (BR338): 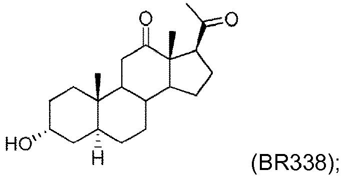 oder folgender Formel (BR053): 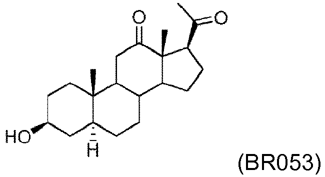 zur Verwendung als Neuroprotektor und/oder als Mittel zur Stimulierung der Proliferation der Nervenzellen.

8. Derivat nach Anspruch 7 zur Behandlung einer Neuropathologie.

9. Derivat zur Behandlung nach Anspruch 8, wobei die Neuropathologie ausgewählt ist aus periphereren und zentralen Neuropathien, chronischem Schmerz, neurodegenerativen Krankheiten und von Nervenentzündungen hervorgerufenen sensorisch-motorischen Defiziten.

10. Derivat zur Behandlung nach Anspruch 9, wobei die Neuropathologie ausgewählt ist aus periphereren und zentralen Neuropathien.

11. Derivat zur Behandlung nach Anspruch 8, wobei die Neuropathologie mit einer Allodynie oder Hyperalgesie in Verbindung steht.

12. Derivat zur Behandlung nach einem der Ansprüche 9 oder 10, wobei die Neuropathologie durch eine Anti-Krebsbehandlung hervorgerufen wird, insbesondere eine chemotherapeutische Behandlung mit Verwendung eines antineoplastischen Mittels, vorteilhaft ausgewählt aus Spindelgiften, interkalierenden Mitteln, Inhibitoren von DNA-Topoisomerasen, Inhibitoren von Tyrosinkinase, Inhibitoren von mTOR, anti-angiogenen monoklonalen Antikörpern, Inhibitoren der DNA-Synthese und -Replikation und Inhibitoren der Polymerisation oder Depolymerisation von Mikrotubuli, und noch vorteilhafter ausgewählt aus Vincristin, Oxaliplatin, Docetaxel, Paclitaxel und anderen Derivaten von Taxol®.

13. Derivat nach einem der Ansprüche 1 bis 5 oder Derivat mit folgender Formel (BR338): 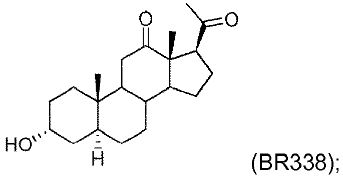 oder folgender Formel (BR053): 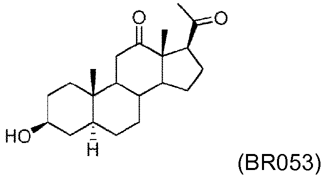 zur Verwendung als Neuroprotektor, insbesondere als Mittel zur Reparatur von Nerven.

14. Derivat nach einem der Ansprüche 1, 2, 3 oder 5 oder Derivat mit folgender Formel (BR338): 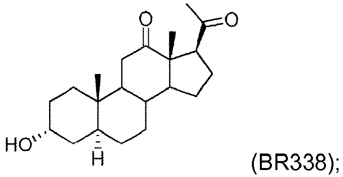 oder folgender Formel (BR053): 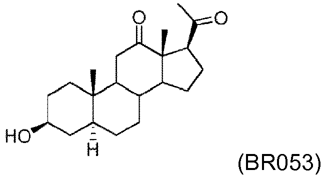 unter Ausschluss des Derivats mit folgender Formel (BR297): 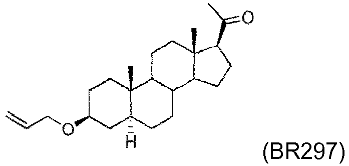 zur Verwendung als Mittel zur Stimulierung der Proliferation von Nervenzellen.

15. Derivat nach Anspruch 4 oder Derivat mit folgender Formel (BR053): 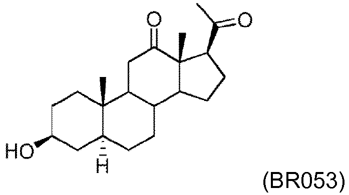 zur Verwendung in einer Dosis von 4 mg des Derivats per kg Körpergewicht alle zwei Tage für einen Patienten, der an einer nach einer in den Ansprüchen 9 bis 10 definierten Neuropathologie leidet.

16. Derivat nach einem der Ansprüche 1 bis 5 oder Derivat mit folgender Formel (BR338): 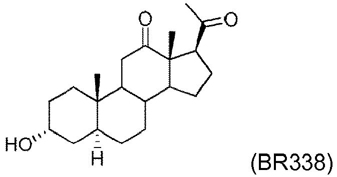 oder folgender Formel (BR053): 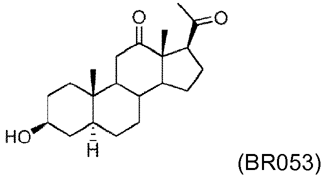 zur Vorbeugung des Zelltods der Neuroblastome, der durch oxidativen Stress induziert wird.

## Claims

1. A derivative of allopregnanolone or 1-((3R,5S,10S,13S,17S)-3-hydroxy-10,1 3-dimethylhexadecahydro-1H-cyclopenta[a]phenanthren-17-yl)ethanone or epiallopregnanolone or 1-((3S,5S,10S,13S,17S)-3-hydroxy-10,13-dimethylhexadecahydro-1H cyclopenta[a]phenanthren-17-yl)ethanone according to the following formula (I): 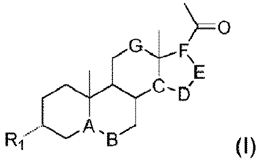 wherein
• **R¹** is a group selected from the 3-alpha or 3-beta hydroxy groups, 3-alpha or 3-beta O-allyl groups, 3-alpha or 3-beta O-propargyl groups, 3-alpha or 3-beta O-glycol groups, 3-alpha or 3-beta O-PEG groups, 3-alpha or 3-beta O-glycol-allyl groups and 3-alpha or 3-beta O-PEG-allyl groups,
• **A** is a carbon atom substituted by an atom selected from 5-H alpha and 5-H beta and **B** is a methylene group; or
**A** and **B** are carbon atoms forming a 5,6-double bond;
• C is a carbon atom substituted by an atom selected from 14-H alpha, 14-H beta, 14-alpha OH group and 14-beta OH and **D** is a methylene group; or
**C** and **D** are carbon atoms forming a 14,15-double bond;
• **F** is a carbon atom substituted by an atom selected from 17-H alpha and 17-H beta and **E** is a methylene group or a carbon atom substituted by a group selected from 16-alkyl-alpha, 16-alkyl-beta, 16-OR²-alpha and 16-OR²-beta, wherein R² is a group selected from allyl, propargyl, glycol, PEG, glycol-allyl, PEG-allyl, or
**F** is a carbon atom substituted by a group selected from 17-alkyl-alpha, 17-alkyl-beta, 17-OR²-alpha and 17-OR²-beta, wherein R² is a group selected from allyl, propargyl, glycol, PEG, glycol-allyl, PEG-allyl, and **E** is a methylene group or a carbon atom substituted by group selected from 16-alkyl-alpha, 16-alkyl-beta, 16-OR²-alpha and 16-OR²-beta, wherein R² is a group selected from allyl, propargyl, glycol, PEG, glycol-allyl, PEG-allyl, or
**E** and **F** are involved in an epoxy cycle or a cyclopropyl and are selected from 16,17-epoxy-alpha, 16,17-epoxy-beta, 16,17-methylene-alpha and 16,17-methylene-beta; or
**E** and **F** are carbon atoms forming a 16,17-double bond;
• **G** is a carbonyl, a methylene or a carbon atom substituted by a 12-OR³-alpha or 12-OR³-beta group wherein R³ is a H atom or a group selected from the acetyl, alkyl and aryl groups,
R³ may be selected from C₁-C₆alkyl, benzyl, *p*-methoxybenzyl, benzoyl, tigloyl, angeloyl, 2,2,2-trichloroethoxycarbonyl, o-aminobenzoyl, nicotinoyl, 2-methylbutyryl, isovaleryl, cinnamoyl, coumaroyl, o-hydroxybenzoyl and anthraniloyl,
excluding (i) the molecule of formula (BR053) below: 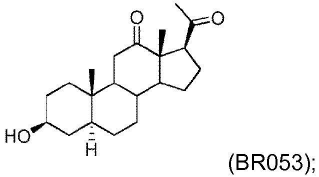 and
(ii) the molecule of formula (BR338) below:

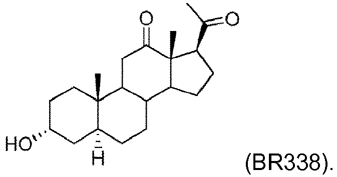

2. Derivative according to claim 1, wherein
• **R¹** is a group selected from the 3-alpha or 3-beta O-allyl groups, the 3-alpha or 3-beta O-propargyl groups, the 3-alpha or 3-beta O-glycol groups, the 3-alpha or 3-beta O-PEG groups, the 3-alpha or 3-beta O-glycol-allyl groups and 3-alpha or 3-beta O-PEG-allyl groups,
• **A** is a carbon atom substituted by an atom selected from 5-H alpha and 5-H beta and **B** is a methylene group; or
**A** and **B** are carbon atoms forming a 5,6-double bond;
• **C** is a carbon atom substituted by an atom selected from 14-H alpha, 14-H beta, 14-alpha OH and 14-beta OH and **D** is a methylene group; or
**C** and **D** are carbon atoms forming a 14,15-double bond;
• **F** is a carbon atom substituted by an atom selected from 17-H alpha and 17-H beta, and **E** is a methylene group or a carbon atom substituted by a group selected from 16-alkyl-alpha, 16-alkyl-beta, 16-OR²-alpha and 16-OR²-beta, wherein R² is a group selected from allyl, propargyl, glycol, PEG, glycol-allyl, PEG-allyl, or
**F** is a carbon atom substituted by a group selected from 17-alkyl-alpha, 17-alkyl-beta, 17-OR²-alpha and 17-OR²-beta, wherein R² is a group selected from allyl, propargyl, glycol, PEG, glycol-allyl, PEG-allyl, and **E** is a methylene group or a carbon atom substituted by a group selected from 16-alkyl-alpha, 16-alkyl-beta, 16-OR²-alpha and 16-OR²-beta, wherein R² is a group selected from allyl, propargyl, glycol, PEG, glycol-allyl, PEG-allyl, or
**E** and **F** are involved in an epoxy cycle or a cyclopropyl and are selected from 16,17-epoxy-alpha, 16,17-epoxy-beta, 16,17-methylene-alpha and 16,17-methylene-beta; or
**E** and **F** are carbon atoms forming a 16,17-double bond; and
• **G** is a carbonyl, a methylene or a carbon atom substituted by a 12-OR³ alpha or 12-OR³ beta group wherein R³ is a H atom or a group selected from the acetyl, alkyl and aryl groups,
R³ may be selected from C₁-C₆alkyl, benzyl, *p*-methoxybenzyl, benzoyl, tigloyl, angeloyl, 2,2,2-trichloroethoxycarbonyl, o-aminobenzoyl, nicotinoyl, 2-methylbutyryl, isovaleryl, cinnamoyl, coumaroyl, o-hydroxybenzoyl and anthraniloyl.

3. Derivative according to claim 2, **characterized in that** it is a derivative of formula (I) wherein **R¹** is a group selected from the 3-beta O-allyl, 3-beta O-propargyl, 3-beta O-glycol, 3-beta O-PEG, 3-beta O-glycol-allyl and 3-beta O-PEG-allyl groups and/or **G** is a methylene.

4. Derivative according to any one of claims 1 to 3 with the following formula (BR297):

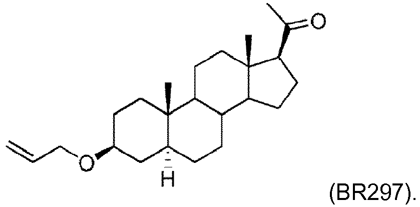

5. Derivative according to any one of claims 1 to 3 with the following formula (BR351):

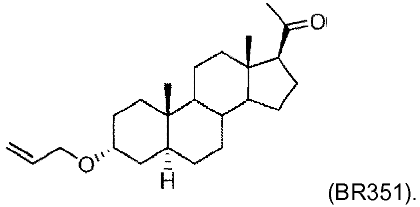

6. Derivative according to any one of claims 1 to 5 for use as medicament.

7. Derivative according to any one of claims 1 to 5 or derivative of the following formula (BR338): 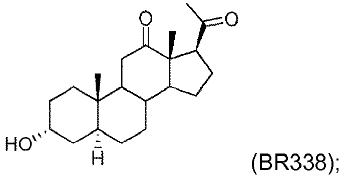 or of the following formula (BR053): 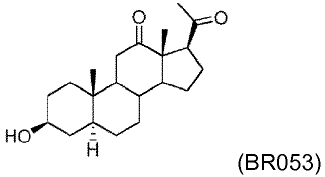 for use as neuroprotective agent and/or as nerve cell proliferation stimulating agent.

8. Derivative according to claim 7 for use in the treatment of a neuropathology.

9. Derivative for use in the treatment according to claim 8, wherein the neuropathology is selected from peripheral and central neuropathies, chronic pain, neurodegenerative diseases and sensory-motor deficits induced by neuroinflammation.

10. Derivative for use in the treatment according to claim 9, wherein the neuropathology is selected from peripheral and central neuropathies.

11. Derivative for use in the treatment according to claim 8, wherein the neuropathology is linked to an allodynia or hyperalgesia.

12. Derivative for use in the treatment according to one of claims 9 or 10, wherein the neuropathy is induced by an anticancer treatment, in particular a chemotherapeutic treatment using an antineoplastic agent advantageously selected from spindle poisons, intercalating agents, topoisomerase DNA inhibitors, tyrosine kinase inhibitors, mTOR inhibitors, anti-angiogenic monoclonal antibodies, inhibitors of DNA synthesis and replication, and microtubule polymerization or depolymerization inhibitors, still more advantageously selected from vincristine, oxaliplatin, docetaxel, paclitaxel and other derivatives of Taxol®.

13. Derivative according to any one of claims 1 to 5 or derivative of the following formula (BR338): 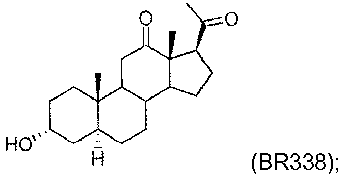 or the following formula (BR053): 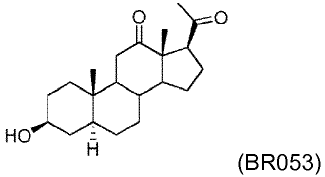 for use as neuroprotective agent, in particular neurorepair agent.

14. Derivative according to any one of claims 1, 2, 3 or 5 or derivative of the following formula (BR338): 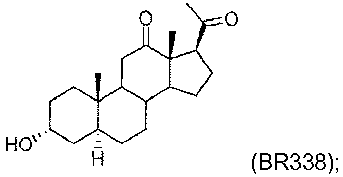 or the following formula (BR053): 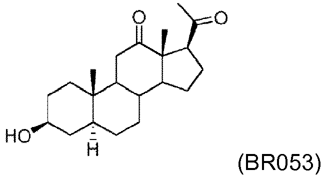 excluding the derivative of the following formula (BR297): 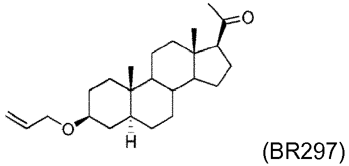 for use as nerve cell proliferation stimulating agent.

15. Derivative according to claim 4 or derivative of the following formula (BR053): 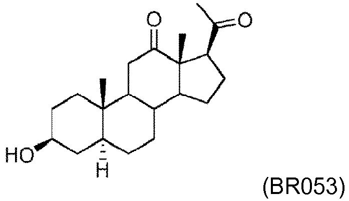 for use at a dose of 4 mg of said derivative per kg of body weight, every other day, in a patient suffering from a neuropathology as defined according to any one of claims 9 to 10.

16. Derivative according to any one of claims 1 to 5 or derivative of the following formula (BR338): 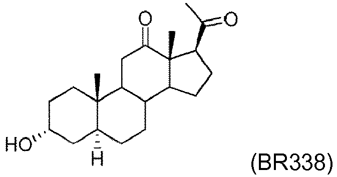 or the following formula (BR053): 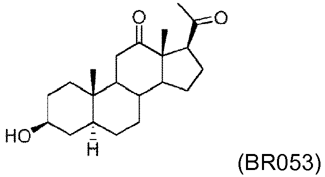 to prevent neuroblastoma cell death induced by oxidative stress.
